# EUROPEAN PATENT SPECIFICATION

(11) **EP 0 687 303 B1**
(45) Date of publication and mention of the grant of the patent: **20.11.2002**
(21) Application number: 94910787.4
(22) Date of filing: 01.03.1994
(51) Int. Cl.: C12N 15/82, C12N 15/29, A01H 5/00, A01H 5/10

(54) **INCREASE OF THE LEVEL OF METHIONIN IN PLANT SEEDS BY EXPRESSION OF 10KD ZEIN FROM CORN**
ERHÖHUNG DES METHIONIN-GEHALTES IN PFLANZENSAMEN DURCH EXPRESSION VON 10KD ZEIN AUS MAIS
AUGMENTATION DU CONTENU EN METHIONINE DANS LES SEMANCES DES PLANTES PAR L'EXPRESSION DE LA 10KD ZEINE DU MAIS

(30) Priority: 02.03.1993 US 29339
(43) Date of publication of application: 20.12.1995
(73) Proprietor: E.I. DU PONT DE NEMOURS AND COMPANY, Wilmington Delaware 19898 (US)
(72) Inventor: WANDELT, Christine, Irmgard, Ardentown, DE 19810 (US)
(74) Representative: Grant, Anne Rosemary
(86) International application number: US9402175
(87) International publication number: WO94020628

(56) References cited:
- WO-A-91/10725
- WO-A-91/13993
- WO-A-92/14822
- MOL CELLS, vol.2, no.3, 1992 pages 297 - 302 PARK, J. W., ET AL. 'Promoter sequence of soybean glycinin gene regulates seed-specific expression in transgenic tobacco plant'
- TRENDS IN BIOTECHNOLOGY, vol.8, no.6, June 1990 pages 156 - 160 ALTENBACH, S.B., ET AL. 'Manipulation of methionine-rich protein genes in seeds'
- J. CELL. BIOCHEM. SUPPL., vol.16F, 1992 page 225 KIRIHARA, J.A., ET AL. 'A transgenic corn line with altered levels of a high-methionine storage protein'
- PLANT MOLECULAR BIOLOGY., vol.18, 1992, DORDRECHT, THE NETHERLANDS. pages 235 - 245 ALTENBACH, S.B., ET AL. 'Accumulation of a Brazil nut albumin in seeds of transgenic canola results in enhanced levels of the seed protein methionine'
- HOFFMAN ET AL.: 'Synthesis and protein body deposition of maize 15-kd zein in transgenic tobacco' EMBO JOURNAL vol. 6, 1987, pages 3213 - 3221
- WILLIAMSON, ET AL.: 'The synthesis of a 19 kilodalton zein protein in transgenic petunia plants' PLANT PHYSIOLOGY vol. 88, 1988, pages 1002 - 1007
- OHTANI ET AL.: 'Normal and lysine-containing zeins are unstable in transgenic tobacco' PLANT MOLECULAR BIOLOGY vol. 16, 1991, pages 117 - 128

### SEQUENCE LISTING

(1) GENERAL INFORMATION:
   (i) APPLICANT:
      (A) NAME: E. I. DU PONT DE NEMOURS AND COMPANY
      (B) STREET: 1007 MARKET STREET
      (C) CITY: WILMINGTON
      (D) STATE: DELAWARE
      (E) COUNTRY: UNITED STATES OF DELAWARE
      (F) POSTAL CODE (ZIP): 19898
      (G) TELEPHONE: 302-992-4931
      (H) TELEFAX: 302-773-0164
      (I) TELEX: 6717325
   (ii) TITLE OF INVENTION: IMPROVED FEEDCROPS ENRICHED IN SULFUR AMINO ACIDS AND METHODS FOR IMPROVEMENT
   (iii) NUMBER OF SEQUENCES: 9
   (iv) COMPUTER READABLE FORM:
      (A) MEDIUM TYPE: DISKETTE, 3.50 INCH, 1.0 MB
      (B) COMPUTER: IBM
      (C) OPERATING SYSTEM: MICROSOFT WINDOWS
      (D) SOFTWARE: MICROSOFT WORD, V2.0C
   (v) CURRENT APPLICATION DATA:
      (A) APPLICATION NUMBER: BB-1045-A
   (vii) PRIOR APPLICATION DATA:
      (A) APPLICATION NUMBER: 08/129,721
      (B) FILING DATE: SEPTEMBER 30,1993
(2) INFORMATION FOR SEQ ID NO:1:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (iii) HYPOTHETICAL: No
   (iv) ANTI-SENSE: No
   (ix) FEATURE:
      (A) NAME/KEY: SM56
      (B) LOCATION: 1..30
      (D) OTHER INFORMATION: /note= "synthetic oligomer"
   (xi) SEQUENCE DESCRIPTION: SEQ ID NO:1:
   ATGGCAGCCA AGATGCTTGC ATTGTTCGCT 30
(2) INFORMATION FOR SEQ ID NO:2:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (iii) HYPOTHETICAL: No
   (iv) ANTI-SENSE: No
   (ix) FEATURE:
      (A) NAME/KEY: CFC88
      (B) LOCATION: 1..30
      (D) OTHER INFORMATION: /note= "synthetic oligomer"
   (xi) SEQUENCE DESCRIPTION: SEQ ID NO:2:
   TTCTATCTAG AATGCAGCAC CAACAAAGGG 30
(2) INFORMATION FOR SEQ ID NO:3:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (iii) HYPOTHETICAL: No
   (iv) ANTI-SENSE: No
   (ix) FEATURE:
      (A) NAME/KEY: OCW11
      (B) LOCATION: 1..18
      (D) OTHER INFORMATION: /note= "SYNTHETIC OLIGOMER"
   (xi) SEQUENCE DESCRIPTION: SEQ ID NO:3:
   GCTGCAACAG CAGTTACC 18
(2) INFORMATION FOR SEQ ID NO:4:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (iii) HYPOTHETICAL: No
   (iv) ANTI-SENSE: No
   (ix) FEATURE:
      (A) NAME/KEY: OCW12
      (B) LOCATION: 1..23
      (D) OTHER INFORMATION: /note= "SYNTHETIC OLIGOMER"
   (xi) SEQUENCE DESCRIPTION: SEQ ID NO:4:
   CGCCACTATC ATGACCCATA TTC 23
(2) INFORMATION FOR SEQ ID NO:5:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (iii) HYPOTHETICAL: No
   (iv) ANTI-SENSE: No
   (ix) FEATURE:
      (A) NAME/KEY: OCW47
      (B) LOCATION: 1..31
      (D) OTHER INFORMATION: /note= "SYNTHETIC OLIGOMER"
   (xi) SEQUENCE DESCRIPTION: SEQ ID NO:5:
   CAACCCTTTG TTGGATCCGC ATTCTAGATA G 31
(2) INFORMATION FOR SEQ ID NO:6:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (iii) HYPOTHETICAL: No
   (iv) ANTI-SENSE: No
   (ix) FEATURE:
      (A) NAME/KEY: OCW70
      (B) LOCATION: 1..19
      (D) OTHER INFORMATION: /note= "SYNTHETIC OLIGOMER"
   (xi) SEQUENCE DESCRIPTION: SEQ ID NO:6:
   GATCAGAAAA AGACGAACT 19
(2) INFORMATION FOR SEQ ID NO:7:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (iii) HYPOTHETICAL: No
   (iv) ANTI-SENSE: No
   (ix) FEATURE:
      (A) NAME/KEY: OCW71
      (B) LOCATION: 1..19
      (D) OTHER INFORMATION: /note= "SYNTHETIC OLIGOMER"
   (xi) SEQUENCE DESCRIPTION: SEQ ID NO:7:
   TCTTTTTCTG CTTGAGATC 19
(2) INFORMATION FOR SEQ ID NO:8:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (iii) HYPOTHETICAL: No
   (iv) ANTI-SENSE: No
   (ix) FEATURE:
      (A) NAME/KEY: OCW98
      (B) LOCATION: 1..19
      (D) OTHER INFORMATION: /note= "SYNTHETIC OLIGOMER"
   (xi) SEQUENCE DESCRIPTION: SEQ ID NO:8:
   CCATTGGGTA CCATGAACC 19
(2) INFORMATION FOR SEQ ID NO:9:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (iii) HYPOTHETICAL: No
   (iv) ANTI-SENSE: No
   (ix) FEATURE:
      (A) NAME/KEY: OCW99
      (B) LOCATION: 1..19
      (D) OTHER INFORMATION: /note= "SYNTHETIC OLIGOMER"
   (xi) SEQUENCE DESCRIPTION: SEQ ID NO:9:
   CATCATGCTC GGCAAGACC 19

## Description

### TECHNICAL FIELD

This invention concerns methods for improving human food and animal feed by transformation of plant cells with novel gene constructs to yield increased levels of sulfur-rich amino acids.

### BACKGROUND OF THE INVENTION

Human food and animal feed derived from many grains are deficient in the sulfur amino acids, methionine and cysteine, which are required in the animal diet. In corn, the sulfur amino acids are the third most limiting amino acids, after lysine and tryptophan, for the dietary requirements of many animals. The use of soybean meal, which is rich in lysine and tryptophan, to supplement corn in animal feed diets is limited by the low sulfur amino acid content of the legume. As a result, synthetic sources of methionine need to be added to provide nutritional value to feed rations. Thus, an increase in the sulfur amino acid content of either corn or soybean would improve the nutritional quality of these mixtures and reduce the need for further supplementation through addition of more expensive methionine. Similarly the use of rapeseed (Brassica napus) meal as an animal feed ingredient would be enhanced by increasing the sulfur amino acid content of the seeds.

The amino acid content of seeds is determined primarily by the storage proteins which are synthesized during seed development and which serve as a major nutrient reserve following germination. The quantity of protein in seeds varies from about 10% of the dry weight in cereals to 20-40% of the dry weight of legumes. In many seeds the storage proteins account for 50% or more of the total protein.

Efforts to improve the sulfur amino acid content of crops through plant breeding have met with limited success on the laboratory scale and no success commercially. A mutant corn line which had an elevated whole-kernel methionine concentration was isolated from corn cells grown in culture by selecting for growth in the presence of inhibitory concentrations of lysine plus threonine (Phillips et al., Cereal Chem., 62:213-218 (1985)). However, agronomically-acceptable cultivars have not yet been derived and commercialized from this line.

Because of their abundance, plant seed storage proteins were among the first proteins to be isolated. Only recently, however, have the amino acid sequences of some of these proteins been determined with the use of molecular genetic techniques. These techniques have also provided information about the genetic signals that control the seed-specific expression and the intracellular targeting of these proteins.

A number of sulfur-rich plant seed storage proteins have been identified and their corresponding genes have been isolated. A gene in corn for a 15 kD zein protein containing 11% methionine and 5% cysteine (Pedersen et al., J. Biol. Chem., 261:6279-6284 (1986)) and a gene for a 10 kD zein protein containing 22% methionine and 3% cysteine have been isolated (Kirihara et al., Mol. Gen. Genet., 21:477-484 (1988); Kirihara et al., Gene, 71:359-370 (1988)). From rice a gene coding for a 10 kD seed prolamin containing 19% methionine and 10% cysteine has been isolated (Masumura et al, Plant Mol. Biol., 12:123-130 (1989)). A high sulfur zein (HSZ) seed storage protein, related but distinct in structure to the 10 kD zein protein, was described by Chui et al. ((1992) W092/14822).

There have been several reports on the expression of seed storage protein genes in transgenic plants. The high sulfur 2S albumin from Brazil nut protein has been expressed in rapeseed (Brassica napus) seeds at a level that resulted in an up to 33% increase in the level of methionine of the salt-extractable seed protein fraction (Altenbach et al., Plant Mol. Biol., 18:235-245 (1992)).

To date, there are no reports of the transfer of the monocotyledonous 10 kD zein gene into and its expression in dicotyledonous plant species such as tobacco, Brassica napus, soybean, sunflower, Arabidopsis, etc.

In order to increase the sulfur amino acid content of seeds it would be useful to develop a method to express a gene coding for a seed storage protein that is rich in the sulfur-containing amino acid, methionine, resulting in high levels of methionine in the seeds of transformed plants. It would be desirable for the storage protein to be compatible with those of the target crop plant and thus have no detrimental effect on seed development. Furthermore, it would be desirable that the protein come from a source that is generally regarded as safe for human food and animal feed. Crop plants obtained by this method would be valuable as sources of sulfur amino acid enriched meal.

### SUMMARY OF THE INVENTION

The subject matter of the invention includes an isolated chimeric gene comprising the following elements operably linked in a 5' to 3' orientation: (a) a dicotyledonous seed-specific promoter, (b) an intracellular localization sequence (c) the maize 10 kD zein coding region without the addition of an ER retention signal, and (d) a 3' non-coding region: wherein said chimeric gene causes an increase in total seed methionine in plant cells in which the gene is expressed as compared to untransformed plant cells; with the proviso that the seed-specific promoter is not the glycinin promoter. Preferred 5' seed specific regulatory sequences are a bean β-phaseolin promoter, more specifically a bean β-phaseolin promoter comprising the bean β-phaseolin 5' untranslated leader region and having the 410 bp of nucleic acid sequence located directly upstream of the bean β-phaseolin transcription initiation site. The preferred 3' seed-specific regulatory sequence is a bean β-phaseolin 3' non-coding region. The preferred intracellular localization sequence for use in the chimeric gene is an amino-terminal signal peptide sequence, with the most preferred being the 10 kD zein amino-terminal signal peptide sequence.

The subject matter of the invention includes a dicotyledonous plant transformed with at least one copy of a chimeric gene comprising the following elements operably linked in a 5' to 3' orientation; (a) a dicotyledonous seed-specific promoter, (b) an intracellular localisation sequence, (c) the maize 10 kD zein coding region without the addition of an ER retention signal, and (d) a 3' non-coding sequence, wherein said chimeric gene causes an increase in total seed methionine in plants in which the gene is expressed as compared to untransformed plant cells, with the preferred plant being a rapeseed or soybean plant. Seeds obtained from the plants are also provided.

The subject matter of the invention includes a method for increasing the sulfur amino acid content of dicotyledonous plants comprising:
(a) transforming a dicotyledonous cell with a chimeric gene comprising the following elements operably linked in a 5' to 3' orientation: (a) a dicotyledonous seed-specific promoter, (b) an intracellular localization sequence, (c) the maize 10 kD zein coding region without the addition of an ER retention signal, and (d) a 3' non-coding region: wherein said chimeric gene causes an increase in total seed methionine in plant cells in which the gene is expressed as compared to untransformed plant cells;
(b) growing fertile, sexually mature plants from the transformed plant cell of step (a); and
(c) selecting progeny seed from the fertile plants of step (b) for increased levels of total seed methionine relative to dicotyledonous plants of the same type as those transformed in step (a) but not containing the chimeric gene.

In a preferred embodiment, the method encompasses increases in the level of total seed methionine in rapeseed relative to seed from untransformed rapeseed from 29% to 232% as compared with Brassica napus (cv. Westar) as untransformed control with 0.76 grams methionine per 100 grams of defatted seed meal.

With the method of the invention rapeseed plants are obtained wherein the polypeptide accumulates to levels from 1% to 8% of total extractable seed protein as 10 kD zein.

### BRIEF DESCRIPTION OF THE DRAWINGS AND SEQUENCE DESCRIPTIONS

The invention can be more fully understood from the following detailed description, the accompanying drawings, and the Sequence Descriptions which form a part of this application.

SEQ ID NOS: 1 and 2 were used in Example 1 to PCR amplify the 10 kD zein coding region from genomic maize DNA.

SEQ ID NO: 3 was used in Example 1 to carry out the mutagenesis of the 10 kD zein gene.

SEQ ID NO: 4 was used in Example 1 to introduce a BspHI site at the signal peptide recognition site of the 10 kD zein gene by site-directed in vitro mutagenesis.

SEQ ID NO: 5 was used in Example 3 to introduce a BamHI site 11 nucleotides upstream of the 10 kD zein translation termination codon by site-directed in vitro mutagenesis

SEQ ID NOS: 6 and 7 in Example 3 represent the oligonucleotide pair coding for the ER retention signal that was inserted at the carboxy-terminus of the 10 kD zein gene.

SEQ ID NOS: 8 and 9 were used in Example 4 for the analysis of plants transformed with 10 kD zein gene constructs.

Figure 1 shows schematically the seed specific expression cassettes pCW108 and pCW109 that were used in Example 3 for the construction of chimeric genes for expression of 10 kD zein in Brassica napus.

Figure 2 shows a summary of the chimeric 10 kD zein gene constructs that were used in Example 4 to transform Brassica napus plants

Figure 3 shows the results of the amino acid analysis of mature seeds of transformed Brassica napus plants.

Figure 4 presents the results of a single seed amino acid analysis of transgenic Brassica napus plant CW189-12(2).

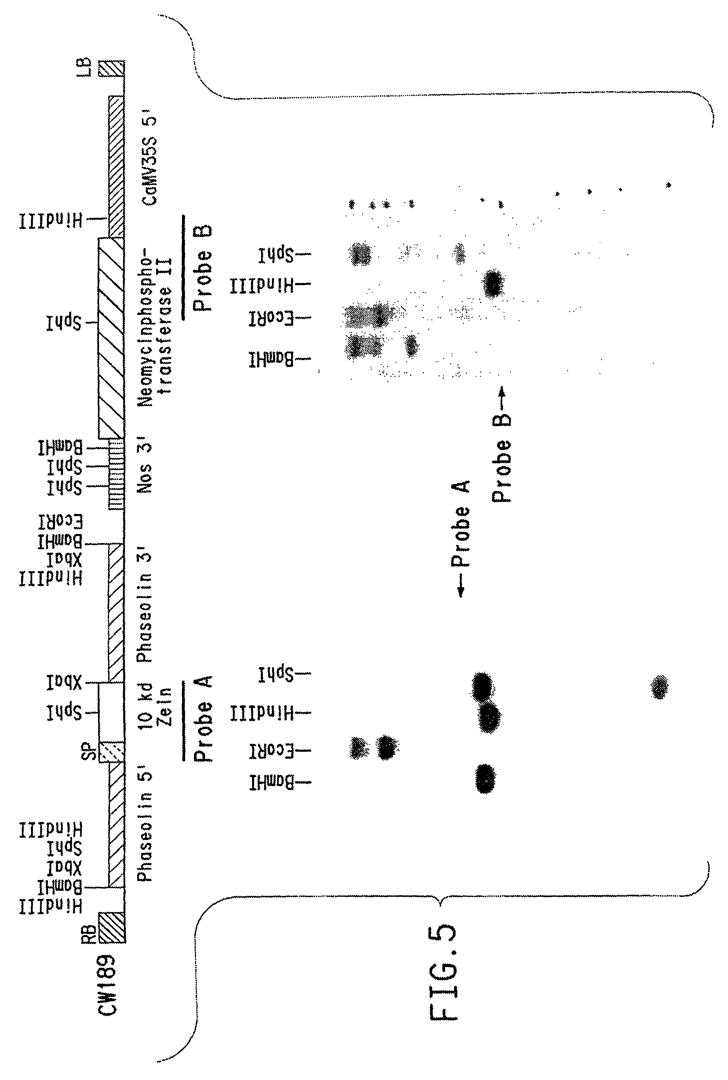

Figure 5 presents the results of a Southern analysis described in Example 7

Figure 6 shows a map of gene construct pCW157 which is described in Example 8.

### DETAILED DESCRIPTION OF THE INVENTION

The present invention describes a novel chimeric gene comprising the monocotyledonous 10 kD zein coding region and a dicotyledonous seed-specific promoter region. The gene is capable of causing an increase in total seed methionine when expressed in a transgenic dicotyledonous plant and thus improves the nutritional quality of the seeds, particularly of rapeseed, sunflower, soybean and other legumes.

The increase in methionine content of the seed will be determined by: (1) the level of expression of the chimeric gene in the transformed crop, which depends, in part, upon the seed-specific expression signals used and on the number of copies of the gene inserted, (2) the percentage of methionine residues in the seed storage protein coding region, (3) the stability of the introduced protein in the seed of the transformed crop plant, which depends, in part, upon its proper processing, intracellular targeting, assembly into higher-order structures in some cases, and ability to withstand dessication, and (4) the compatibility of the introduced protein with the native seed proteins of the transformed crop. A transgenic rapeseed (Brassica napus cv. Westar) plant which distinguishes itself from an untransformed Westar plant by an increase in total methionine levels in mature seeds of 102% illustrates the effect of the chimeric gene in dicotyledonous plants. A comparable effect based on the described chimeric gene might be achieved in transgenic soybean seed as well due to the similarity of rapeseed and soybean embryos as oil and protein storage organs.

### DEFINITIONS

In the context of this disclosure, a number of terms shall be utilized.

As used herein, the term "nucleic acid" refers to a large molecule which can be single-stranded or double-stranded, composed of monomers (nucleotides) containing a sugar, phosphate and either a purine or pyrimidine. A "nucleic acid fragment" or "nucleic acid sequence" is a fraction of a given nucleic acid molecule. As used herein the term "gene" refers to a nucleic acid fragment that expresses a specific protein, including regulatory sequences preceding (5' non-coding, 5' untranslated leader) and following (3' non-coding) the coding region. "Chimeric" gene refers to a gene comprising heterogeneous regulatory and coding sequences.

"Coding sequence" refers to a DNA sequence that codes for a specific protein and excludes the non-coding sequences.Depending on the protein the coding region can include intracellular localization sequences, such as an amino-terminal signal peptide, such as a signal peptide and a vacuolar targeting sequence, such as a signal peptide and an Endoplasmic Reticulum retention sequence.

"RNA transcript" refers to the product resulting from RNA polymerase-catalyzed transcription of a DNA sequence. When the RNA transcript is a perfect complementary copy of the DNA sequence, it is referred to as the primary transcript or it may be a RNA sequence derived from posttranscriptional processing of the primary transcript and is referred to as the mature RNA. "Messenger RNA" (mRNA) refers to the RNA that is without introns and that can be translated into protein by the cell. "cDNA" refers to a double-stranded DNA that is complementary to and derived from mRNA. "Transcription initiation site" refers to a nucleotide in the 5' flanking region of a gene that specifies the initiation of the RNA polymerase-catalyzed transcription of that gene.

"Regulatory sequences" refer to nucleotide sequences located upstream (5'), within, and/or downstream (3') to a coding sequence, which control the transcription and/or expression of the coding sequences, potentially in conjunction with the protein biosynthetic apparatus of the cell. These nucleotide sequences include a promoter sequence, a 5' untranslated leader sequence, a transcription termination sequence, and a polyadenylation sequence.

"Promoter" refers to a DNA sequence in a gene, usually upstream (5') to its coding sequence, which controls the expression of the coding sequence by providing the recognition for RNA polymerase and other factors required for proper transcription. A promoter may also contain DNA sequences that are involved in the binding of protein factors which control the effectiveness of transcription initiation in response to physiological or developmental conditions. A promoter may also contain enhancer elements.

An "enhancer" is a DNA sequence which can stimulate promoter activity. It may be an innate element of the promoter or a heterologous element inserted to enhance the level and/or tissue-specificity of a promoter. "Organ-specific" or "development-specific" promoters (e.g., "seed-specific promoter") as referred to herein are those that direct gene expression almost exclusively in specific organs, such as leaves or seeds, or at specific development stages in an organ, such as in early or late embryogenesis, respectively.

The term "expression", as used herein, is intended to mean the production of the protein product encoded by a gene. "Overexpression" or "overproduction" refers to the production of a gene product in transgenic organisms that exceeds levels of production in normal or non-transformed organisms.

The "3' non-coding sequences" refers to the DNA sequence portion of a gene that contains a transcription termination signal, polyadenylation signal, and any other regulatory signal capable of affecting mRNA processing or gene expression. The polyadenylation signal is usually characterized by affecting the addition of polyadenylic acid tracts to the 3' end of the mRNA precursor.

The "5' non-coding sequences" refers to the DNA sequence portion of a gene that contains a promoter sequence and a 5' untranslated leader sequence.

The "5' untranslated leader sequence" refers to that DNA sequence portion of a gene between the promoter and coding sequence that is transcribed into RNA and is present in the fully processed mRNA upstream (5') of the translation start codon. The 5' untranslated leader sequence may affect processing of the primary transcript to mRNA, mRNA stability or translation efficiency.

"Intracellular localization sequence " refers to a nucleotide sequence that encodes an intracellular targeting signal. An "intracellular targeting signal" is an amino acid sequence which is translated in conjunction with a protein and directs it to a particular sub-cellular compartment. "Signal peptide" (SP) refers to the amino terminal extension of a polypeptide, which is translated in conjunction with the polypeptide forming a precursor peptide and which is required for its entrance into the secretory pathway. The term "signal peptide sequence" refers to a nucleotide sequence that encodes the signal peptide. "Endoplasmic reticulum (ER) retention signal" refers to a carboxy-terminal extension of a polypeptide, which is translated in conjunction with the polypeptide and causes a protein that enters the secretory pathway to be retained in the ER. "ER retention signal sequence" (ER) refers to a nucleotide sequence that encodes the ER retention signal. "Vacuolar targeting signal" refers to an amino-terminal, internal or carboxy-terminal peptide sequence, which is translated in conjunction with the polypeptide and causes a protein that enters the secretory pathway to be transported through the Golgi compartment to the vacuole. "Vacuolar targeting sequence" refers to the nucleotide sequence that encodes the vacuolar targeting signal.

"Meal" refers to the defatted, hexane-extracted fraction of the seed.

"Seed components" refers to fractions of the seed and products obtained from the processing of such seed including meal, oil, and protein.

"Total seed methionine" refers to the amount of methionine that can be extracted from either non-defatted or from defatted seed meal and includes free methionine and protein-bound methionine.

"Total seed protein" refers to the amount of protein that can be extracted from defatted or non-defatted seed meal using aqueous and/or alcoholic solutions.

"Transformation" herein refers to the transfer of a foreign gene into the genome of a host organism and its genetically stable inheritance. Examples of methods of plant transformation include Agrobacterium-mediated transformation and accelerated-particle or "gene gun" transformation technology.

"Multiple copies" refers to the stable integration of a foreign gene into the genome of a host organism at a single locus in tandem or inverted arrays or the integration of a foreign gene into the genome of a host organism at multiple loci as single copy and/or in tandem or inverted arrays.

Recombinant DNA technology offers the potential for increasing the sulfur amino acid content of crop plants. Particularly useful technologies are: (a) methods for the molecular cloning and in vitro manipulation of genes (see Sambrook et al., Molecular Cloning: a Laboratory Manual, Cold Spring Harbor Laboratory Press (1989)), (b) introduction of genes via transformation into agriculturally-important crop plants such as soybean (Chee et al., Plant Physiol., 91:1212-1218 (1989); Cluisrou et al., Proc. Nat. Acad. Sci U.S.A., 86:7500-7504 (1989); Hinchee et al., Biotechnology, 6:915-922 (1989); EPO publication 0301 749 A2) and rapeseed (De Block et al., Plant Physiol., 91:694-701 (1989)), and (c), seed-specific expression of introduced genes in transgenic plants (see Goldberg et al., Cell 56:149-160 (1989); Thompson and Larkins, BioEssays, 10:108-113 (1989)).

### Isolation of the 10 kD Zein Gene and its Expression in E.coli

The aim of the present invention was to increase the sulfur content of rapeseed (Brassica napus) soybean. sunflower, and other dicotyledonous seeds via introduction of the 10 kD zein gene with a methionine content of 22%, which belongs to the prolamin group of maize seed storage proteins and accumulates in protein bodies of the maize endosperm cells. To date, there are no reports of the transfer of the monocotylodonous 10 kD zein gene into and its expression in dicotyledonous plant species such as tobacco, Brassica napus, etc. Park et al (Mol. Cells, 1992, 2(3), p297-302) observed low levels of transcription of soybean glycinin gene using 10 kD zein as a reporter in tobacco, but failed to note any expression of that gene. The 10 kD zein gene has been shown to be overexpressed in a maize line selected via breeding (Kirihara et al., Mol. Gen. Genet., 211:477-484 (1988)) and to be expressed in a maize line where it has been introduced via genetic engineering (see publication WO91/10725). There are only few reports on the efficient expression of the maize seed storage proteins, the zeins, in seeds of transgenic dicotyledonous plants. The so-called alpha zeins which comprise the 19 kD and 22 kD class of the maize prolamins accumulate to 0.005% of total seed protein in transgenic tobacco when placed under the control of the dicotyledonous bean seed specific phaseolin promoter (Ohtani et al., Plant Mol. Biol., 16:117-128 (1991); Williamson et al., Plant Phys., 88:1002-1007 (1988)). When placed under the control of the Cauliflower Mosaic Virus 35S promoter neither a 19 kD nor a 22 kD zein protein could be detected in embryos of mature tobacco seeds, although both proteins were present in endosperm tissue at levels up to 0.1 % of ethanol-soluble proteins (Schemthaner et at.(1988) EMBO J. 7:1249-1255). In contrast, the 15 kD zein, another representative of the endosperm specific maize prolamins, under the control of the dicotyledonous bean seed specific phaseolin promoter can be expressed to levels of 1.6% of total tobacco seed protein (Hoffinan et al., EMBO J., 6:3213-3221 (1987)). The gene of this invention, the 10 kD zein gene does not show any similarity on amino acid and nucleotide sequence level with the 15 kD zein, but shares sequence similarity in its signal peptide with the alpha zeins (Kirihara et al., Gene, 71:359-370 (1988)) and co-localizes with the alpha zeins at the core of the endosperm protein body after its synthesis on the rough endoplasmic reticulum (Lending and Larkins, The Plant Cell, 1:1011-1023 (1989); Esen and Stetler, American J. Botany, 79:243-248 (1992)).

The previously published maize 10 kD zein gene (Kirihara et al., Gene, 71:359-370 (1988)) has been cloned from a maize genomic DNA library. The preferred constructs for expression of the 10 kD zein gene in E. coli was a signal peptide deletion mutant under the control of the bacteriophage T7 promoter (Rosenberg et al., Gene, 56:125-135 (1987)) as described in Examples 1 and 2. The 10 kD zein with its own amino-terminal signal peptide could be used as well instead of the signal peptide deletion mutant. Due to the very hydrophobic nature of the 10 kD zein inclusion bodies formed in the E. coli cells overexpressing the 10 kD zein. 10 kD which accumulated in the inclusion bodies could easily be extracted with 70% 2-propanol in the presence of beta-mercaptoethanol to yield almost pure 10 kD zein protein. This protein isolation and purification scheme was used to isolate 10 kD zein in sufficient quantities to immunize rabbits and goat to generate polyclonal antibodies in rabbit and goat. Many other microbial expression vectors have been described in the literature. One skilled in the art could make use of any of these to construct 10 kD zein expression vectors. These 10 kD zein expression vectors could then be introduced into appropriate microorganisms via transformation to provide a system for high level expression of 10 kD zein.

### Chimeric Gene Constructs For Expression of 10 kD Zein in Dicotyledonous Plants

The expression of foreign genes in plants is well established (De Blaere et al., Meth. Enzymol., 143:277-291 (1987)). Proper levels of expression of 10 kD zein may require the use of different chimeric expression cassettes utilizing different promoters. Particularly preferred among the higher plants and the seeds derived from them are rapeseed, soybean, pea and other legumes, sunflower, etc. The source of the promoter chosen to drive the expression of the coding sequence is not critical as long as it has sufficient transcriptional activity to accomplish the invention by expressing 10 kD zein in the desired host tissue. Preferred promoters are those that allow expression of the protein, specifically in seeds. Examples of seed-specific promoters include, but are not limited to the promoters of seed storage proteins. The seed storage proteins are strictly regulated, being expressed almost exclusively in seeds in a highly organ-specific and stage specific manner (Higgins et al., Ann. Rev. Plant Physiol., 35:191-221 (1984); Goldberg et al., Cell, 56:149-160 (1989); Thompson et al., BioEssays, 10:108-113 (1989)). Moreover, different seed storage proteins may be expressed at different stages of seed development.

There are currently numerous examples for seed-specific expression of seed storage protein genes in transgenic dicotyledonous plants. These include genes from dicotyledonous plants for bean β-phaseolin (Sengupta-Gopalan et al., Proc. Natl. Acad. Sci. USA. 82:3320-3324 (1985); Hoffman et al., Plant Mol. Biol., 11:717-729 (1988)). bean lectin (Voelker et al., EMBO J., 6:3571-3577 (1987)), soybean lectin (Okamuro et al.. Proc. Natl. Acad. Sci. USA, 83:8240-8244 (1986)), soybean kunitz trypsin inhibitor (Perez-Grau et al., Plant Cell, 1:095-1109 (1989)), soybean β-conglycinin (Beachy et al., EMBO J., 4:3047-3053 (1985); Barker et al., Proc. Natl. Acad. Sci. USA, 85:458-462 (1988); Chen et al., EMBO J., 7:297-302 (1988); Chen et al., Dev. Genet., 10:112-122 (1989); Naito et al., Plant Mol. Biol., 11:109-123 (1988)), pea vicilin (Higgins et al., Plant Mol. Biol., 11:683-695 (1988)), pea convicilin (Newbigin et al., Planta, 180:461 (1990)), pea legumin (Shirsat et al., Mol. Gen. Genetics, 215:326 (1989)); rapeseed napin (Radke et al., Theor. Appl. Genet., 75:685-694 (1988); Stayton et al., Aus. J. Plant Phys., 18:507-517 (1991)). Moreover, promoters of seed-specific genes, operably linked to heterologous coding sequences in chimeric gene constructs, also maintain their temporal and spatial expression pattern in transgenic plants. Such examples include Arabidopsis thaliana 2S seed storage protein gene promoter to express enkephalin peptides in Arabidopsis and B. napus seeds (Vandekerckhove et al., Bio/Technology, 7:929-932 (1989)), bean lectin and bean β-phaseolin promoters to express luciferase (Riggs et al., Plant Sci., 63:47-57 (1989)).

Of particular use in the expression of the nucleic acid fragment of the invention will be the promoters from several extensively characterized soybean seed storage protein genes such as those for the Kunitz trypsin inhibitor (Jofuku et al., Plant Cell, 1:1079-1093 (1989); Perez-Grau et al., Plant Cell, 1:1095-1109 (1989)), glycinin (Nielson et al., Plant Cell, 1:313-328 (1989)), β-conglycinin (Harada et al., Plant Cell, 1:415-425 (1989)); and from the extensively characterized bean seed storage protein gene for phaseolin (Doyle et al., J. Biol. Chem., 261:9228-9238 (1986)). The β-phaseolin promoter has been characterized in more detail regarding regulatory domains required for tissue and developmental specific expression (Burow et al., Plant J., 2:537-548 (1992); Bustos et al., Plant Cell, 1:839-853 (1989); Bustos et al., EMBO J., 10:1469-1479 (1991); Kawagoe and Mural. Plant J., 2:927-936 (1992)).

Especially preferred among the seed storage protein promoters is a version of the bean β-phaseolin promoter encompassing 410 bp upstream of the transcription initiation site and a version of the soybean β-conglycinin α'-subunit encompassing 550 bp upstream of the transcription initiation site (see publication WO91/13993 and Doyle et al., J. Biol. Chem., 261:9228-9238 (1986)). One can also envision a modified β-phaseolin promoter with its silencer regions deleted (Bustos et al., EMBO J., 10:1469-1479 (1991)) as a promoter which might confer expression levels up to 100% higher than the unmodified full length β-phaseolin promoter. In tobacco transformed with β-phaseolin promoter deletion constructs a minimum of 795 bp upstream of the transcription initiation site is necessary for 100% promoter activity in seeds; a promoter fragment encompassing only 418 bp upstream of the transcription initiation site will give 20% promoter activity in transgenic tobacco seeds (Bustos et al., EMBO J., 10:1469-1479 (1991)).

The introduction of enhancers or enhancer-like elements into other promoter constructs will also provide increased levels of primary transcription for a 10 kD zein gene to accomplish the invention. These would include viral enhancers such as that found in the 35S promoter (Odell et al., Plant Mol. Biol., 10:263-272 (1988)), enhancers from the opine genes (Fromm et al., Plant Cell, 1:977-984 (1989)), or enhancers from any other source that result in increased transcription when placed into a promoter operably linked to the nucleic acid fragment of the invention. Of particular importance is the DNA sequence element isolated from the gene for the α'-subunit of β-conglycinin that can confer 40-fold seed-specific enhancement to a constitutive promoter (Chen et al., EMBO J., 7:297-302 (1988); Chen et al., Dev. Genet., 10:112-122 (1989)). One skilled in the art can readily isolate this element and insert it within the promoter region of any gene in order to obtain seed-specific enhanced expression with the promoter in transgenic plants. Insertion of such an element in any seed-specific gene that is expressed at different times than the β-conglycinin gene will result in expression in transgenic plants for a longer period during seed development.

Any 3' non-coding region capable of providing a polyadenylation signal and other regulatory sequences that may be required for the proper expression of the 10 kD zein coding region can be used to accomplish the invention. This would include the 3' end from any storage protein such as the 3' end of the bean β-phaseolin gene, the 3' end of the soybean β-conglycinin gene, the 3' end from viral genes such as the 3' end of the 35S or the 19S cauliflower mosaic virus transcripts, the 3' end from the opine synthesis genes, the 3' ends of ribulose 1,5-bisphosphate carboxylase or chlorophyll a/b binding protein, or 3' end sequences from any source such that the sequence employed provides the necessary regulatory information within its nucleic acid sequence to result in the proper expression of the promoter/10 kD zein coding region combination to which it is operably linked. There are numerous examples in the art that teach the usefulness of different 3' non-coding regions (for example, see Ingelbrecht et al., Plant Cell, 1:671-680 (1989)).

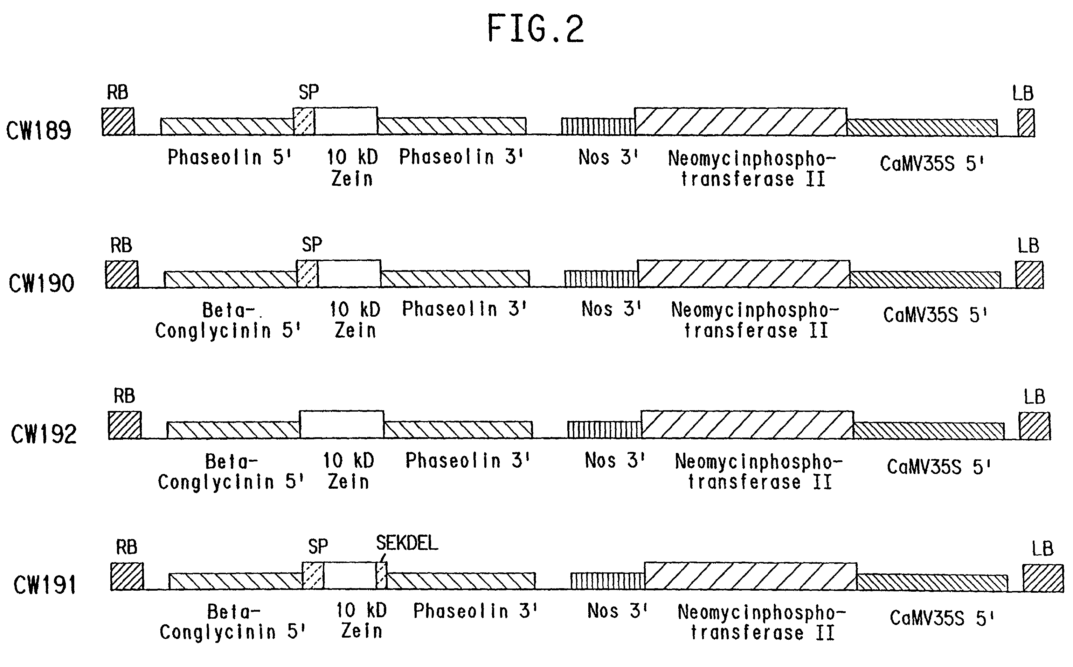

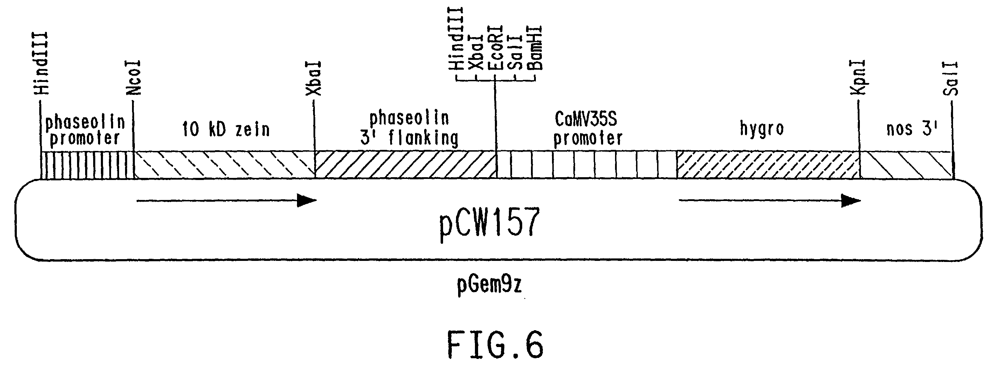

In addition, the efficient expression of 10 kD zein in dicotylodonous hosts might benefit from suitable intracellular localization signals. 10 kD zein is a maize endosperm specific seed storage protein and accumulates in protein bodies that are derived from the endoplasmatic reticulum membrane (Esen and Stetler, American J. Bot., 79:243-248 (1992): Lending et al., Protoplasma, 143:51-62 (1988); Wallace et al., Biochem. Physiol. Planzen. 183:107-115 (1988); Lending and Larins, Plant Cell, 1:1011-1023 (1989)). In contrast, in dicot embryos seed storage proteins such as the soybean or pea seed storage proteins accumulate in protein bodies that are derived from the vacuole (Chrispeels, Annu. Rev. Plant Physiol. Plant Mol. Biol., 42:21-53 (1991); Chrispeels and Raikhel, Cell, 68:613-616 (1992)). The effect of this is that the intracellular localization of seed storage proteins differs significantly in maize endosperm from Brassica napus or soybean embryos. Correct targeting of the 10 kD zein in heterologous hosts might have a significant impact on the stability and thus on the expression levels of this protein. One can envision adding an ER retention signal carboxy-terminal to the 10 kD zein so that it will be retained in the ER and not transported to the vacuole and thus facilitate the accumulation of 10 kD zein in Brassica napus embryos. The invention does not however extend to the inclusion of such sequences. The highly conserved sequence Lys-Asp-Glu-Leu (KDEL) present at the carboxy-terminus of mammalian reticuloplasmins was shown to be necessary for their retention and appears to be sufficient to reduce the secretion of several secretory proteins (Munro and Pelham, Cell, 48:899-907 (1987); Zagouras and Rose, J. Cell. Biol., 109:2633-2640 (1989); Pelham, EMBO J., 7:913-918 (1988); Pellham et al., EMBO J., 7:1757-1762 (1988); Inohara et al, Proc. Natl. Acad. Sci. USA, 86:3564-3568 (1989); Hesse et al., EMBO J., 8:2453-2461 (1989); Tillman et al., EMBO J., 8:2463-2467 (1989); Akasofu et al., Nucleic Acids Res., 17:6733 (1989); Tanaka et al, Plant Mol. Biol., 16:1083-1084 (1991); Denecke et al., EMBO J., 11:2345-2355 (1992)). The addition of the KDEL sequence to the carboxy-terminus of the pea vicilin gene increased its stability in transgenic tobacco leaves by 100 fold (Wandelt et al., Plant J., 2:181-192 (1992)). It may be useful to direct the 10 kD zein to the vacuole of Brassica napus embryo cells by adding a vacuolar targeting signal. Short amino-, carboxy-terminal or internal interspersed amino acid domains that direct proteins to the vacuole have been identified in several plant seed storage proteins (Chrispeels and Raikhel, Cell, 68:613-616 (1992): Bednarek and Raikhel, Plant Mol. Biol., 20:133-150 (1992); Bednarek and Raikhel. Plant Cell, 3:1195-1206 (1991); Holwerda et al., Plant Cell, 4:307-318 (1992): Matsuoka et al., J. Biol. Chem., 265:19750-19757 (1990); Neuhaus et al., Proc. Natl. Acad. Sci. USA. 88:10362-10366 (1991)). Seed storage proteins enter the secretory pathway via an amino-terminal extension, the signal peptide. One could create a 10 kD zein gene lacking the signal peptide which would render the protein cytoplasmic (Chrispeels, Annu. Rev. Plant. Physiol. Plant Mol. Biol., 42:21-53 (1991); Wandelt et al., Plant Molecular Biology, 2:471-478 (1991)). The following two chimeric constructs (see also Example 3) are the preferred constructs of the invention. (1) bean β-phaseolin -410 promoter/10 kD zein/bean (β-phaseolin 3' flanking region; (2) soybean β-conglycinin -550 promoter/10 kD zein/bean β-phascolin 3' flanking region. Constructs (3) soybean β-conglycinin -550 promoter/10 kD zein-SEKDEL/bean β-phaseolin 3' flanking region and (4) soybean β-conglycinin -550 promoter/SPdel-10 kD zein/bean β-phaseolin 3' flanking region illustrate comparative constructs. The constructs are schematically shown in Figure 2. (The abbreviations used in Figure 2 and Figure 6 are as follows: "RB" refers to Right Border; "LB" refers to Left Border; "SP" refers to Signal Peptide; "NOS" refers to Nopaline Synthase": "CaMV" refers to Cauliflower Mosaic Virus; "SEKDEL" refers to Ser-Glu-Lys-Asp-Glu-Leu, ER Retention Signal.)

### Transformation of Dicotyledonous Plants with Chimeric Gene Constructs for Expression of 10 kd Zein

Various methods of introducing a DNA sequence (i.e., of transforming) into eukaryotic cells of higher plants are available to those skilled in the art (see EPO publications 0 295 959 A2 and 0 138 341 A1). Such methods include those based on transformation vectors based on the Ti and Ri plasmids of Agrobacterium spp. It is particularly preferred to use the binary type of these vectors. Ti-derived vectors transform a wide variety of higher plants, including monocotyledonous and dicotyledonous plants, such as soybean, cotton and rape (Pacciotti et al., Bio/Technology. 3:241 (1985); Byrne et al.. Plant Cell, Tissue and Organ Culture, 8:3 (1987); Sukhapinda et al., Plant Mol. Biol.. 8:209-216 (1987); Lorz et al., Mol. Gen. Genet.. 199:178 (1985); Potrykus, Mol. Gen. Genet., 199:183 (1985)).

For introduction into plants the chimeric genes of the invention can be inserted into binary vectors as described in Examples 3-6. The vectors are part of a binary Ti plasmid vector system (Bevan, Nucl. Acids. Res., 12:8711-8720 (1984)) of Agrobacterium turnefaciens. Other transformation methods are available to those skilled in the art, such as direct uptake of foreign DNA constructs see EPO publication 0 295 959 A2), techniques of electroporation (see Fromm et al., Nature (London), 319:791 (1986)) or high-velocity ballistic bombardment with metal particles coated with the nucleic acid constructs (see Klein et al., Nature (London), 327:70 (1987), and see U.S. Pat. No. 4,945,050). Once transformed, the cells can be regenerated by those skilled in the art.

Of particular relevance are the recently described methods to transform foreign genes into commercially important crops, such as rapeseed (see De Block et al., Plant Physiol., 91:694-701 (1989)), sunflower (Everett et al., Bio/Technology, 5:1201 (1987)), soybean (McCabe et al., Bio/Technology, 6:923 (1988); Hinchee et al., Bio/Technology, 6:915 (1988); Chee et al., Plant Physiol., 91:1212-1218 (1989); Christou et al., Proc. Natl. Acad. Sci USA, 86:7500-7504 (1989); EPO Publication 0 301 749 A2; Finer et al., In Vitro Cell. Dev. Biol., 27P:175-182 (1991)), and corn (Gordon-Kamm et al., Plant Cell, 2:603-618 (1990); Fromm et al., Biotechnology, 8:833-839 (1990)).

### Expression of 10 kD Zein in Transgenic Dicotyledonous Plants

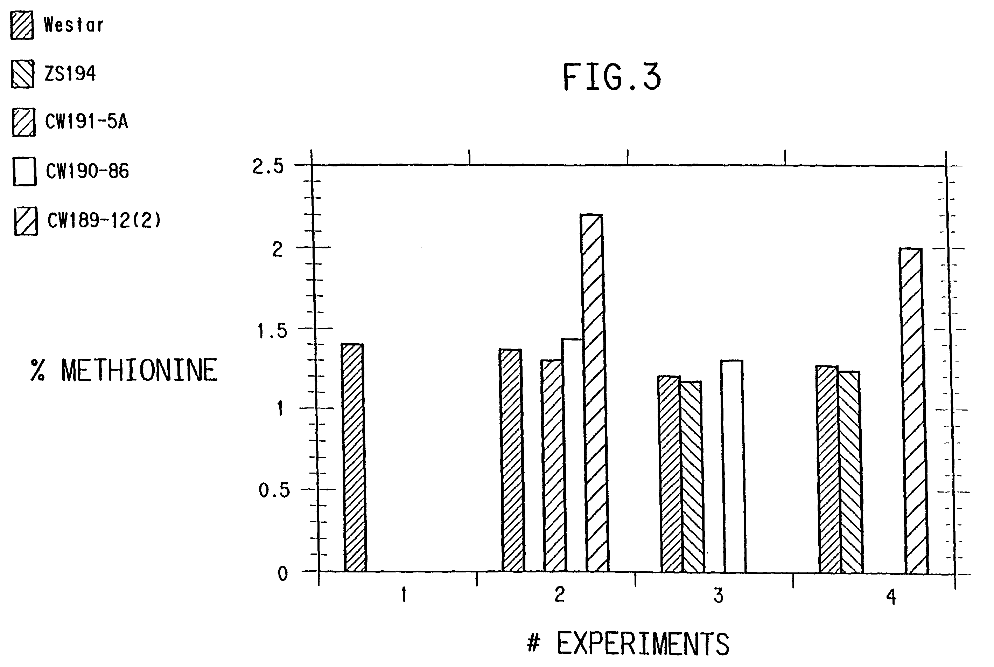

To assay for expression of the chimeric genes in seeds of the transformed plants, the 10 kD zein protein can be extracted, detected and quantitated immunologically by methods known to those skilled in the art. Amino acid analysis methods can be used to measure the increase in total methionine levels due to the expression of the introduced high methionine 10 kD zein gene (see Examples 5 and 6). Using these assays transgenic Brassica napus lines expressing high levels of 10 kD zein were identified. Significant increases in overall methionine levels in mature seeds (via multiple single seed or seed meal analysis) from transgenic Brassica napus lines transformed with 10 kD zein gene constructs were detected. One out of 24 plants showed an increase in total methionine of 102% (heterozygous situation) which corresponds to expression levels of 3.9% of total seed protein; 6 out of 24 plants show expression levels of 10 kD zein of 0.4 to 1% of total seed protein. Seventeen out of 24 plants show expression levels of less than 0.05% of total seed protein. This group of plants is predominantly found in lines that have been transformed with 10 kD zein genes harboring modified targeting signals (signal peptide deletion or ER retention signal addition). 10 kD zein with its own signal peptide accumulates stably in seeds of transformed Brassica napus plants. In a homozygous situation expression levels of almost 8% 10 kD zein of total seed protein might be achieved according to the single seed amino acid analysis (see Figure 3).

Inter-transformant variability in transgenic expression as seen in the above description could be related to the copy number of the introduced genes, the site of integration and other factors. There are no examples known where a clear correlation between transgene copy number and transgene expression has been shown; there are even indications that single copies result in higher expression of the transgene (see Hobbs et al., Plant Mol. Biol., 15:851-864 (1990); and references therein). However, some examples exist wherein the highest expression levels correlate with a high gene copy number. Altenbach et al., Plant Mol. Biol., 13:513-522 (1989); Leisy et al., Plant Mol. Biol., 14:41-50 (1989)). As shown in Example 7, the gene copy number is of importance in achieving high levels of expression in transgenic Brassica napus seeds. The highest expressing plant shows integration of the transgene at at least 2 major loci with a total gene copy number of >5. One can envision constructs harboring the 10 kD zein gene under the control of seed specific promoters in tandem arrays on a binary vector to achieve higher copy numbers of the introduced gene of interest.

Canola meal is widely used in Canada for feeding poultry because of its nutritional value and availability at a price competitive with other protein supplements. The protein content of canola meal ranges from 37 to 38%. Feeding trials on the use of canola meal for broilers usually contain 10 to 20% of canola meal to supplement either wheat and/or com, the principal grain component. Canola meal is relatively high in lysine, an essential amino acid deficient in cereal grains, and methionine, which is beneficial when fed with grains, such as maize, in which these essential amino acids are limiting. Because canola meal is considered equivalent in quality to that of soybean, an increase in the level of lysine and methionine could be an advantage in the market. One can envision the use of canola seed meal from a canola plant with an increased seed methionine content as great as 232% as a significantly improved feed supplement in the poultry industry.

### EXAMPLES

The present invention is further defined in the following Examples, in which all parts and percentages are by weight and degrees are Celsius, unless otherwise stated. It should be understood that these Examples, while indicating preferred embodiments of the invention, are given by way of illustration only.

### EXAMPLE 1

### 10 kD Zein Constructs for Expression in E. Coli

The coding region of the high methionine 10 kD maize seed storage protein or 10 kD zein (for sequence see Kirihara et al., Gene, 71:359-370 (1988)) was isolated from genomic maize DNA (line B85) using the polymerase chain reaction (PCR). Two oligonucleotides flanking the 10 kD zein coding region were synthesized (Applied Biosystems). The PCR reaction was performed using a Perkin-Elmer Cetus kit according to the instructions of the vendor on a thermocycler manufactured by the same company. The PCR reaction product of 450 bp was blunt-ended with Klenow DNA polymerase, cleaved with XbaI (see underlined sequence in oligonucleotide CFC88) and subcloned into the commercially available (Pharmacia) vector pTZ18R linearized with SmaI and XbaI, yielding the plasmid pCC1. The entire coding region was sequenced (United States Biochemicals kit). The sequence deviated from the published coding sequence (Kirihara et al., Gene, 71:359-370 (1988)) in one base pair at nucleotide position 1504 of the published sequence. An A was changed to a G which resulted in the change of amino acid 123 (with the initiator methionine as amino acid 1) from Gln to Arg. It is not known if the detected mutation was generated during the PCR reaction or if this is indeed another allele of the maize 10 kD zein gene. To revert the mutation back to the published sequence the plasmid pCC1 was mutagenized by oligonucleotide-directed site-specific mutagenesis (BioRad kit, Hercules, CA) using oligonucleotide OCW11. The resulting plasmid named pCW116 was sequenced to verify the reversal of the mutation (see underlined nucleotide in SEQ ID NO:3) and used as a basic construct for the chimeric genes presented in Example 3. In a second mutagenesis reaction with plasmid pCC1 and oligonucleotides OCW11 and OCW12 a BspHI site (see underlined nucleotides in SEQ ID NO:4) was created at the signal peptide recognition site (amino acids representing the amino-terminus of the mature 10 kD zein are underlined in SEQ ID NO:4). Sequencing of the resulting plasmid pCW119 confirmed the reversal of the mutation at site 1504 of the published sequence (Kirihara et al., Gene, 71:359-370 (1988)) from 5'-GCAACGGC-3' to 5'-GCAACAGC-3' and the creation of a BspHI site from 5'-CTAGTGCGA-3' to 5'-CTATCATGA-3'. To achieve high levels of expression of the mature 10 kD zein coding sequence in E. coli, the bacterial expression vector pBT430 was used. This vector is a derivative of pET-3a (Rosenberg et al., Gene, 56:125-135 (1987)) which employs the bacteriophage T7 RNA polymerase/T7 promoter system. Plasmid pBT430 was constructed by first destroying the EcoRI and HindIII sites in pET-3a at their original positions. An oligonucleotide adaptor containing EcoRI and HindIII sites was then inserted at the BamHI site of pET-3a. This created pET-3aM with additional unique cloning sites for insertion of genes into the expression vector. Then, the NdeI site at the position of translation initiation was converted to an NcoI site using oligonucleotide-directed mutagenesis. The DNA sequence of pET-3aM in this region, 5'-CATATGG (NdeI site underlined), was converted to 5'-CCCATGG (NcoI site underlined) in pBT430. The BspHI/HindIII fragment of plasmid pCW119 was ligated into pBT430 linearized with NcoI and HindIII (for standard cloning procedures see Sambrook et al., Molecular Cloning: A Laboratory Manual. Cold Spring Harbor Laboratory Press (1989)) resulting in plasmid pCW124. This plasmid carries only the region coding for the mature form of the 10 kD zein under the control of the bacteriophage T7 promoter.

### EXAMPLE 2

### Overexpression of 10 kD zein in E. coli

A 10 L culture of E. coli strain BL21(DE3) (Studier et al., Methods in Enzymology, 185:60-89 (1990)) harboring the plasmid pCW124 was grown in LB medium plus ampicillin (0.1 g/L) at 37°C under standard conditions in a BIOSTAT E (B. Brown Company). At OD₆₀₀ₙₘ of 0.7 the culture was induced with isopropylthio-β-galactoside (IPTG) to a final concentration of 0.4 mM and incubation was continued over night at 37°C with the culture reaching a final OD₆₀₀ₙₘ of 1.0. The volume of the culture medium was reduced to 0.9 L by membrane filtration after which the cells were harvested by a final centrifugation step. The cell pellet was washed twice with 100 mL of 50 mM NaCl, 50 mM Tris-HCl, 1 mM EDTA, pH 7.5 (TEN) and frozen at -20°C. The pellet was thawed, resuspended in 50 mL of TEN including PMSF to a final concentration of 1 mM (TEN+P) and sonicated at 4°C. Cell debris was collected in a spin for 10 min at 10 krpm. The pellet (mainly inclusion bodies) was extracted 3 times with a total of 100 mL 70% 2-propanol, 0.2% β-mercaptoethanol, yielding 700 mg of greater than 95% pure 10 kD zein. Aliquots from all 3 alcoholic fractions of 10 kD zein were subjected to SDS-polyacrylamide gel electrophoresis. The predominant band visible by Coomassie Blue staining was the 10 kD zein. About four milligrams of the purified protein were sent to Hazelton Research Facility, 310 Swampridge Road, Denver, PA 17517, to obtain antibodies raised against 10 kD zein in rabbit and goat.

### EXAMPLE 3

### Chimeric Gene Constructs for Expression of 10 kD Zein in Brassica Napus (cv. Westar)

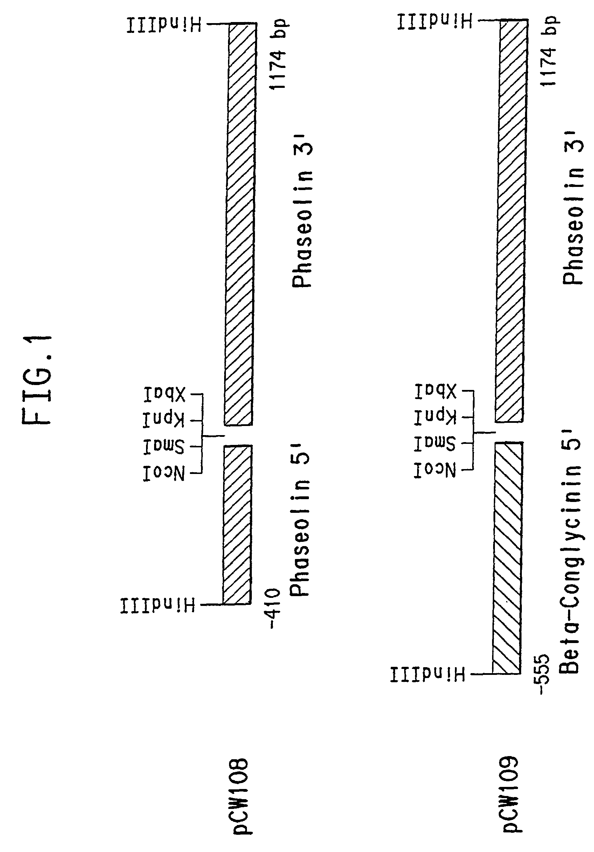

Two seed-specific expression cassettes were used as a basis for chimeric gene constructs for expression of 10 kD zein in Brassica napus. The cassettes are shown schematically in Figure 1. Cassette pCW108 contains 410 basepairs of the bean (Phaseolus vulgaris) phaseolin (β-subunit of the 7S seed storage protein) promoter starting with 5'-TGGTCTTTTGGTTCATG-3' (see sequence in Doyle et al., J. Biol. Chem., 261:9228-9238 (1986) and WO91/13993), the β-phaseolin 5' untranslated leader and the β-phaseolin 3' untranslated region of 1174 base pairs. Cassette pCW109 contains 555 basepairs of the β-conglycinin promoter (α' subunit of the 7S seed storage protein) promoter from soybean (Glycine max), the β-conglycinin 5' untranslated region and 1174 basepairs of the bean β-phaseolin 3' untranslated region. The sequence represents an allele of the published β-conglycinin gene (Doyle et al., J. Biol. Chem., 261:9228-9238 (1986)) due to differences at 27 nucleotide positions, although none of them in a region that has been shown to be important for enhanced and tissue specific expression of this seed storage protein gene. A multiple cloning site (NcoI, SmaI, KpnI and XbaI) in cassettes pCW108 and pCW109 separates the 5' flanking region from the 3' flanking region and allows the convenient insertion of any coding region. The 10 kD zein coding region from plasmid pCW116 was inserted as a NcoI/XbaI fragment into the seed specific expression cassettes pCW108 and pCW109 resulting in the following constructs, which are flanked by unique HindIII restriction sites: (1) pCW126: β-phaseolin 5'-10 kD zein-β-phaseolin 3', and (2) pCW128: β-conglycinin 5'-10 kD zein-β-phaseolin 3'. To facilitate subcloning of the above chimeric constructs into binary Ti based vectors for plant transformation, (1) pCW126 was digested with HindIII, which creates a restriction fragment harboring the β-phaseolin 5'-10 kD zein coding-β-phaseolin 3' chimeric gene and was ligated into pUC1318 (Kay et al., Nucl. Acids Res., 6:2778 (1987)) linearized with HindIII, resulting in plasmid pCW171. (2) Plasmid pCW128 was digested with HindIII and subcloned into the commercially available vector Bluescript SK(-) cut with HindIII resulting in plasmid pCW182. Both constructs pCW171 and pCW182 with their convenient cloning sites enable the ligation of the 10 kD zein seed specific expression cassettes into binary Ti plasmid based vectors (Bevan, Nucl. Acids. Res., 12:8711-8720 (1984)) of Agrobacterium tumefaciens. The vectors contain: (1) the chimeric gene CaMV35S/neomycin phosphotransferase/nopaline synthase (35S:NPTII:nos) as a selectable marker for transformed plant cells (Bevan et al., Nature, 304:184-186 (1983)), (2) the left and right borders of the T-DNA of the Ti plasmid (Bevan, Nucl. Acids. Res., 12:8711-8720 (1984)), (3) the E. coli lacZ α-complementing segment (Vieria and Messing, Gene, 19:259-267 (1982)) with unique restriction endonuclease sites for EcoR I, Kpn I, BamH I and Sal I, (4) the bacterial replication origin from the Pseudomonas plasmid pVS1 (Itoh et al., Plasmid, 11:206-220 (1984)), and (5) the bacterial neomycin phosphotransferase gene from Tn5 (Berg et al., Proc. Natl. Acad Sci. USA, 72:3628-3632 (1975)) as a selectable marker for transformed
A. tumefaciens. Plasmid pCW171 was digested with BamHI yielding the chimeric gene fragment: β-phaseolin 5'-10 kD zein-β-phaseolin 3', which was ligated into the binary vector pZS199 linearized with BamHI resulting in plasmid pCW189 (see Figure 2). Plasmid pCW182 was digested with Sail and EcoRI yielding the chimeric gene β-conglycinin 5'-10 kD zein-β-phaseolin 3', which was ligated into the binary vector pZS198 linearized with Sail and EcoRI, resulting in plasmid pCW190 (see Figure 2). In addition, two targeting mutant constructs of 10 kD zein were created: (1) with a deleted signal peptide sequence, and (2) with an Endoplasmic Reticulum (ER) retention signal sequence added. To obtain the signal peptide deletion mutant plasmid pCW119 (see Example 1) was digested with BspHI and XbaI and the resulting mature 10 kD zein coding sequence was ligated into the seed expression cassette pCW109 which has been linearized with NcoI and XbaI, resulting in plasmid pCW125. To facilitate further cloning of the 10 kD zein coding region under the control of the β-conglycinin promoter, the plasmid pCW125 was digested with HindIII and the β-conglycinin-10 kD zein-β-phaseolin 3' fragment was ligated into the vector pUC1318 linearized with HindIII, yielding the plasmid pCW151. In order to transfer the 10 kD zein signal peptide deletion mutant gene construct into a binary vector the plasmid pCW151 was digested with BamHI and the 10 kD zein chimeric gene was ligated into the binary vector pZS199 linearized with BamHI, resulting in plasmid pCW192 (see Figure 2). To add the ER retention signal sequence SerGluLysAspGluLeu (Pelham, Trends Biochem. Sci., 15:483-486 (1990)) onto the carboxy terminus of 10 kD zein, the following mutagenesis reaction was performed. The plasmid pCC1 (see Example 1) was mutagenized using oligonucleotide OCW11 (see Example 1) and oligonucleotide OCW47. Mutagenesis with OCW47 introduces a BamHI, cleavage site (see underlined nucleotides in SEQ ID NO:5) 11 basepairs upstream of the 10 kD zein translation stop codon. The sequence 5'-GGTGCTGCA-3' is changed into 5'-GGATCCGCA-3'. The resulting plasmid pCW152 was sequenced to verify the introduced mutations and digested with BamHI to allow the insertion of the oligonucleotide pair OCW70 and OCW71, coding for the ER retention signal. The resulting plasmid was pCW173. In the next cloning step the 10 kD zein-SEKDEL mutant was inserted into the seed specific expression cassette pCW109 via NcoI and XbaI, resulting in plasmid pCW180. To ease subcloning into a binary vector the β-conglycinin 5'-10 kD zein-β-SEKDEL-phaseolin 3' chimeric gene was moved from pCW180 as a HindIII fragment into the commercially available vector BluescriptSK(-), resulting in plasmid pCW183. Digestion of pCW183 with Sail and EcoRI allowed ligation of the chimeric β-conglycinin 5'-10 kD zein-SEKDEL-phaseolin 3' gene into the binary vector pZS198, linearized with Sail and EcoRI. The resulting plasmid was pCW191 (see Figure 2). As a summary of the above described chimeric constructs see Figure 2.

### EXAMPLE 4

### Transformation of Brassica napus (cv. Westar) with the chimeric gene constructs pCW189, pCW190, pCW191 and pCW192

The binary vectors containing the chimeric 10 kD zein genes were transferred by tri-parental mating (Ruvkin et al., Nature, 289:85-88 (1981)) to Agrobacterium strain LBA4404/pAL4404 (Hockema et al., Nature, 303:179-180 (1983)).

The transformed Agrobacterium strains were used to inoculate seedling pieces of Brassica napus cultivar "Westar" using the following procedure.

B. napus seeds are sterilized by stirring in 10% Chlorox, 0.1% SDS for thirty min, and then rinsed thoroughly with sterile distilled water. The seeds were germinated on sterile medium containing 30 mM CaCl₂ and 1.5% agar, and grown for six d in the dark at 24°C.

Liquid cultures of Agrobacterium for plant transformation were grown overnight at 28°C in Minimal A medium containing 100 mg/L kanamycin. The bacterial cells were pelleted by centrifugation and resuspended at a concentration of 10⁸ cells/mL in liquid Murashige and Skoog Minimal Organic medium containing 100 µM acetosyringone.

B. napus seedling hypocotyls were cut into 5 mm segments which were immediately placed into the bacterial suspension. After 30 min, the hypocotyl pieces were removed from the bacterial suspension and placed onto BC-12 callus medium containing 100 µm acetosyringone. The plant tissue and Agrobacteria were co-cultivated for three d at 24°C in dim light.

The co-cultivation was terminated by transferring the hypocotyl pieces to BC-12 callus medium containing 200 mg/L carbenicillin to kill the Agrobacteria, and 25 mg/L kanamycin to select for transformed plant cell growth. The seedling pieces were incubated on this medium for three weeks at 24°C under continuous light.

After three weeks, the segments were transferred to BS-48 regeneration medium containing 200 mg/L carbenicillin and 25 mg/L kanamycin. Plant tissue was subcultured every two weeks onto fresh selective regeneration medium, under the same culture conditions described for the callus medium. Putatively transformed calli grew rapidly on regeneration medium; as calli reached a diameter of about 2 mm, they were removed from the hypocotyl pieces and placed on the same medium lacking kanamycin.

Shoots began to appear within several weeks after transfer to BS-48 regeneration medium. As soon as the shoots formed discemable stems, they were excised from the calli, transferred to MSV-1A elongation medium, and moved to 16:8-hour photoperiod at 24°C.

Once shoots had elongated several intemodes, they were cut above the agar surface and the cut ends were dipped into ROOTONE. Treated shoots were planted directly into wet METRO-MIX 350 soiless potting medium in an 8" standard pot. The pots were covered with plastic bags which were removed when the plants were clearly growing -- usually after about ten days.

Plants were grown in a growthroom at 20°C under a 16:8-hour photoperiod. They were watered three times daily with 0.5X Hoagland's Solution until they began flowering. At flowering, nutrients were discontinued and the plants were watered daily as needed to prevent wilting. Finally, watering was discontinued when pods began to yellow. Some plants were moved to a greenhouse at flowering; in the greenhouse, the temperature ranged from 20 to 25°C, and the photoperiod was 14:10 hours.

When plants began to flower, they were covered with mesh pollen-containment bags to prevent outcrossing. Self-pollination was facilitated by shaking the plants several times each day. Seeds derived from self-pollinations were harvested about three months after planting.
Minimal A Bacterial Growth Medium
   Dissolve in distilled water:
      10.5 g potassium phosphate, dibasic
      4.5 g potassium phosphate, monobasic
      1.0 g ammonium sulfate
      0.5 g sodium citrate, dihydrate
   Make up to 979 mLs with distilled water
   Autoclave
   Add 20 mLs filter-sterilized 10% sucrose
   Add 1 mL filter-sterilized 1 M MgSO₄
Brassica Callus Medium BC-12
   Per liter:
      Murashige and Skoog Minimal Organic Medium (MS salts,
      100 mg/L i-inositol, 0.4 mg/L thiamine; GIBCO #510-3118)
      30 g sucrose
      18 g mannitol
      1 mg/L 2,4-D
      3 mg/L kinetin
      0.6% agarose
      pH 5.8
Brassica Regeneration Medium BS-48
   Murashige and Skoog Minimal Organic Medium Gamborg B5
   Vitamins (SIGMA #G1019)
   10 g glucose
   250 mg xylose
   600 mg MES
   0.4% agarose
   pH 5.7
Filter-sterilize and add after autoclaving:
   2.0 mg/L zeatin
   0.1% mg/L IAA
Brassica Shoot Elongation Medium MSV-1A
   Murashige and Skoog Minimal Organic Medium Gamborg B5
   Vitamins
   10 g sucrose
   0.6% agarose
   pH 5.8
Table 1 gives an overview over the regenerated plants. A plant was considered sterile if the mature seed harvest totaled less than 40 seeds. Brassica napus leaf genomic DNA isolated from transgenic plants (DNA isolation procedure modified from Shure et al., Cell, 35:225-233 (1986)) was analyzed by PCR for the presence of the 10 kD zein coding region using oligonucleotides CW98 and OCW99, which amplify a 212 base pair fragment spanning the amino-terminal half of the 10 kD zein coding region.

**TABLE 1**

| | | | |
|---|---|---|---|
| CW189 β-phaseolin 5'-10 kD zein-β-phaseolin 3' | | | |
| CW190 β-conglycinin 5'-10 kD zein-β-phaseolin 3' | | | |
| CW191 β-conglycinin 5'-SPdel-10 kD zein-β-phaseolin 3' | | | |
| CW192 β-conglycinin 5'-10 kD zein-SEKDEL-β-phaseolin 3' | | | |

| TRANSFORMANT | LINE | GENE | FERTILITY |
|---|---|---|---|
| CW189- | 1A | + | sterile |
| | 4 | + | sterile |
| | 12(2) | + | |
| | 52 | + | sterile |
| | 56 | - | |
| | 60 | + | |
| | 66(2) | + | |
| CW190- | 2 | - | |
| | 12 | + | |
| | 24 | + | sterile |
| | 34 | - | |
| | 43 | + | |
| | 57(2) | + | |
| | 81 | + | |
| | 82 | + | sterile |
| | 86 | + | |
| | 91(2) | + | |
| CW190- | 92 | + | |
| CW191- | 2 | - | |
| | 5A | + | |
| | 6 | + | sterile |
| | 73 | + | sterile |
| CW192- | 1A | + | |
| | 5A | - | |
| | 11 | - | |
| | 13 | + | sterile |
| | 14 | + | |
| | 19 | + | sterile |
| | 28 | + | |
| | 59 | - | |
| | 61 | - | |
| | 62 | + | |
| | 72 | - | |
| | 80 | + | |
| | 83 | + | |
| | 86 | + | |
| | 89B | + | sterile |
| | 118 | + | sterile |
| | 119 | - | |

### EXAMPLE 5

### Western Analysis of Brassica napus (cv. Westar) Transformant Lines CW189, CW190, CW191 and CW192

To assay for expression of the chimeric 10 kD zein genes in seeds of transformed Brassica napus plants, protein was extracted as follows. Fifteen mature seeds were harvested from each transformant, ground in a mortar with pestel under liquid nitrogen; the fine canola seed meal was transferred to eppendorf tubes and extracted twice with 1 mL of hexane, the solvent clinging to the meal was dried at 65°C for 10 min. The partially defatted seed meal was extracted once with 300 µL 50 mM Tris-HCl pH 6.8, 2 mM EDTA, 1% SDS, 1 mM PMSF (TES+P) and spun at 15 krp at room temperature for 5 min. The remaining pellet was extracted twice with 450 µL 70% 2-propanol, 1 mM PMSF, 1% β-mercaptoethanol, the alcohol fractions were combined and dried in a speed-vac. The dry pellet was dissolved in 50 µL TES, the protein concentration was determined (BioRad kit, Hercules, CA) against BSA as protein standard. Ten µg of total protein were subjected to SDS-PAGE. Proteins were electrophoretically blotted from the gel onto nitrocellulose membrane in a Tris-glycine buffer omitting the methanol. The membranes were incubated with rabbit 10 kD zein-antibodies (see Example 2) at a 1:1000 dilution of the rabbit serum using a standard protocol (BioRad). The secondary antibody was donkey anti-rabbit IgG conjugated to horseradish peroxidase (Amersham) at a 1:3000 dilution. The specific antibody reaction was visualized by a chemiluminescence reaction (Amersham). Both aqueous and alcohol fractions of the extraction of Brassica seed meal of transformants was analyzed via Western. 10 kD zein is only present in the alcohol fraction. The signal peptide of 10 kD zein is processed in all cases analyzed so far. The mobility of the 10 kD zein on SDS-polyacrylamide gels is comparable to the 10 kD zein overexpressed in E. coli (see Example 2). As estimated from the Western, the expression level of 10 kD zein in the transformants analyzed ranges from 4% of total seed protein for plant CW189-12(2) to less than 0.005% of total seed protein for plant CW192-1A. Table 2 illustrates the results of the Western analysis.

**TABLE 2**

| | | |
|---|---|---|
| CW189 phaseolin 5'-10 kD zein-phaseolin 3' | | |
| CW190 β-conglycinin 5'-10 kD zein-phaseolin 3' | | |
| CW191 β-conglycinin 5'-SPdel-10 kD zein-phaseolin 3' | | |
| CW192 β-conglycinin 5'-10 kD zein-SEKDEL-phaseolin 3' | | |

| TRANSFORMANT | LINE | WESTERN |
|---|---|---|
| CW189- | 12(2) | ++++++ |
| | 60 | +++ |
| | 66(2) | +++ |
| CW190- | 12 | +++++ |
| | 43 | - |
| | 57(2) | +++++ |
| | 81 | ++++ |
| | 86 | ++++ |
| CW190- | 91(2) | ++++ |
| | 92 | ++++ |
| CW191- | 5A | ++ |
| CW192- | 1A | +++ |
| | 14 | ++ |
| | 28 | +++ |
| | 62 | ++ |
| | 80 | ++ |
| | 83 | ++ |
| | 86 | +++ |
| | 118 | ++ |
| | 119 | nd |

### EXAMPLE 6

### Amino Acid Analysis of Brassica napus (cv. Westar) Transformants CW189-12(2)

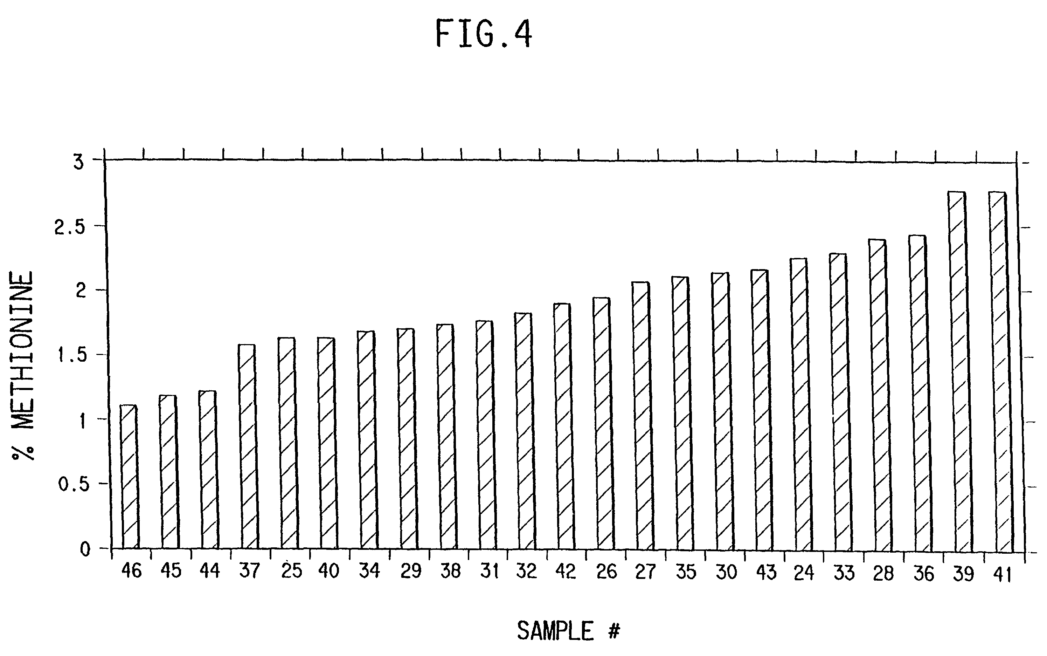

To measure the amino acid composition of mature transgenic brassica seeds, (1) 15 mature seeds were ground to a fine powder under liquid nitrogen in a mortar with pestel and hexane extracted twice; approximately two milligrams of partially defatted canola seed meal was subjected to amino acid analysis, or (2) one single mature seed (approx. 5 mg) was crushed and subjected to amino acid analysis: the samples were hydrolyzed in 6 N hydrochloric acid, 0.4% β-mercaptoethanol under nitrogen for 24 h at 110-120°C; aliquots of samples (1) or (2) were run on a Beckman Model 6300 amino acid analyzer using post-column ninhydrin detection. The amino acid analysis results of analyzed transgenic plants are shown in Figures 3 and 4. Values are given as percent methionine of all amino acids analyzed (excluding cysteine and tryptophane).

Table 3 gives the results of the amino acid analysis of the control plants Westar and ZS194, a binary vector-only canola transformant (refer to binary Ti plasmid based vectors described in Example 3), and CW189-12(2), the highest expressing canola transformant for 10 kD zein. As indicated by A, B or C (column 1), 15 individual seeds from one transformant were ground to seed meal, defatted and analyzed as described above. Given are the values (columns 3 and 4), the mean and standard deviation (columns 5 and 6) for three aliquots (column 2) of the same seed meal batch. The methionine content is expressed as nmole (column 3) and grams per 100 grams seed meal (column 4). Given as well are the total amino acid yields in µmole (column 8), excluding tryptophane and cysteine, and based on that the calculated amount of extracted protein (column 9).
Columns 10 and 11 illustrate the efficiency of the extraction based on the expected protein content of canola meal of 37%.

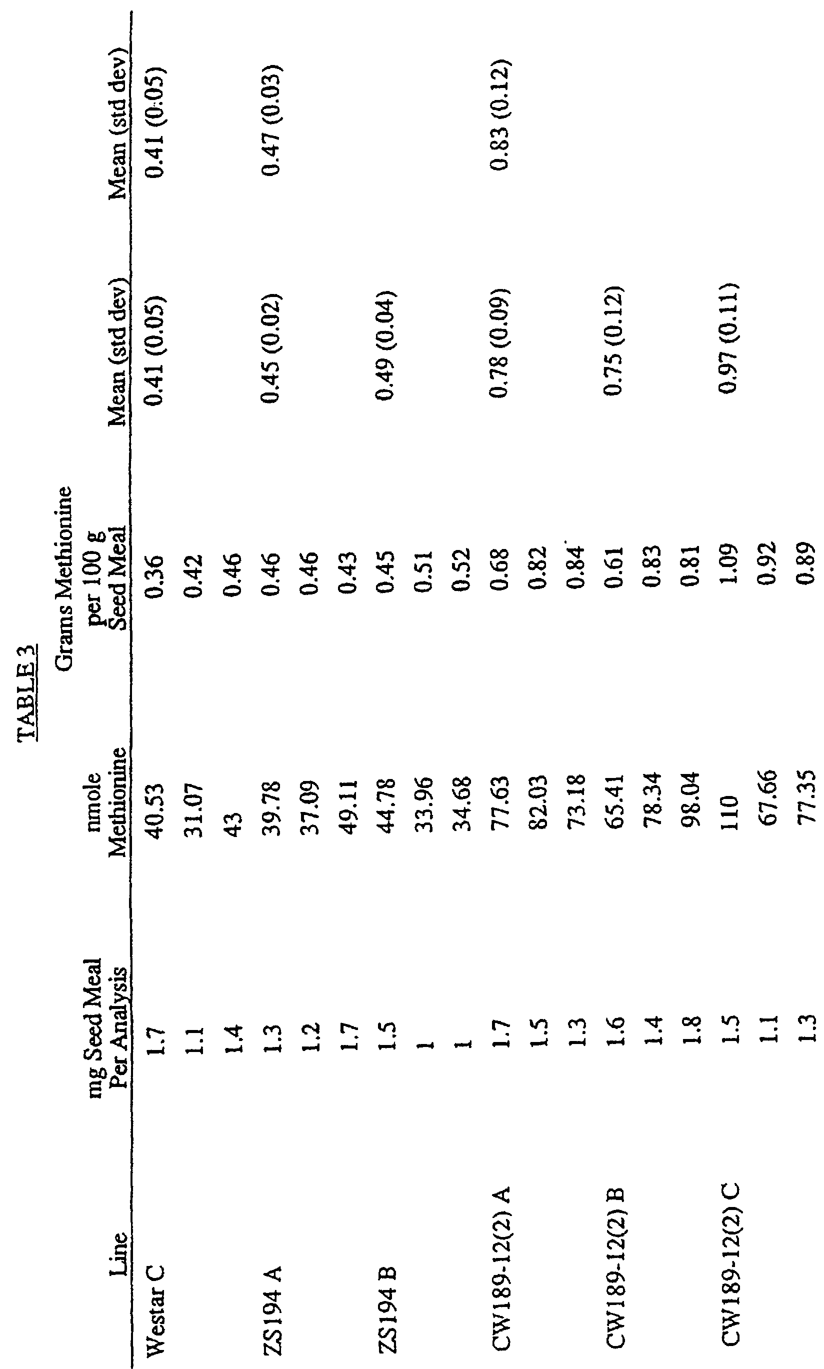

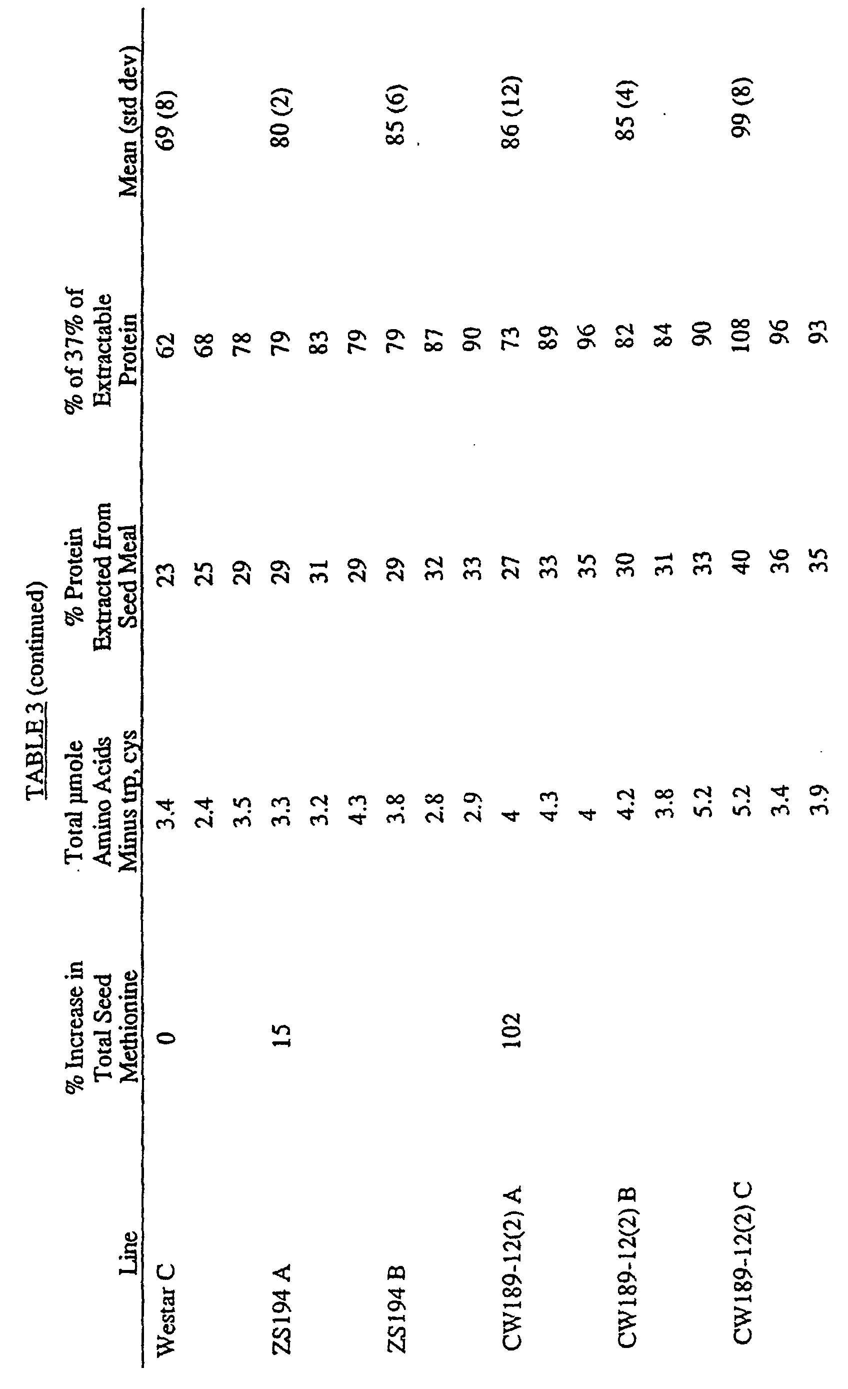

The methionine content of Brassica napus (cv. Westar) is described in the literature as 0.76 grams per 100 grams defatted seed meal. The methionine values obtained and described in this invention are the values based on the amino acid analysis method given in this example and based on the growth conditions (see Example 4) of the analyzed transformed plants. Given in Example 5 is the expression level of 10 kD zein in plant CW189-12(2) as approximately 4% 10 kD zein of total extractable seed protein. One can calculate as shown in Table 5 the expected increase in total seed methionine, based on this expression level and the mole percent methionine of the mature 10 kD zein protein (see Table 4).

**TABLE 4**

| Amino Acid Composition of Mature 10 kD Zein Polypeptide | | |
|---|---|---|
| Residue | Number | Mole Percent |
| Alanine | 7 | 5.4 |
| Cysteine | 5 | 3.9 |
| Aspartic acid | 1 | 0.8 |
| Phenylalanine | 5 | 3.9 |
| Glycine | 4 | 3.1 |
| Histidine | 3 | 2.3 |
| Isoleucine | 4 | 3.1 |
| Leucine | 15 | 11.6 |
| Methionine | 28 | 21.7 |
| Asparagine | 3 | 2.3 |
| Proline | 20 | 15.5 |
| Glutamine | 15 | 11.6 |
| Serine | 8 | 6.2 |
| Threonine | 5 | 3.9 |
| Valine | 5 | 3.9 |
| Tyrosine | 1 | 0.8 |
| Total | 129 | 100 |

**TABLE 5**

| Grams Methionione per 100 grams of seed meal | % 10 kD Zein of Total Seed Protein | % Increase in Total Seed Methionine |
|---|---|---|
| 0.76 | 0 | 0 |
| 1.20 | 2 | 58 |
| 1.64 | 4 | 116 |
| 2.08 | 6 | 174 |
| 2.52 | 8 | 232 |

As shown in Table 3, column 7, the increase in total seed methionine for transformant CW189-12(2) of 102% is in good agreement with the calculated 116% increase in total seed methionine (given in Table 5) based on the literature value for a total canola seed meal methionine content of 0.76 g methionine per 100 g of seed meal.

The single seed analysis shown in Figure 4 shows part of the possible range of total seed methionine levels (expressed as % methionine of analyzed amino acids) in a segregating seed population of 20 seeds from the transgenic canola plant CW189-12(2). One can envision based on the values given in Table 5 that in a progeny plant homozygous for the 10 kD zein gene(s) the total seed methionine increase will be as high as 232% assuming an expression of the 10 kD zein at levels of 8% of total seed protein.

### EXAMPLE 7

### Southern Analysis of Brassica napus (cv. Westar) Transformant CW189-12(2)

Genomic DNA was extracted from Brassica napus transformant leaves (see also Example 4), digested with HindIII, EcoRI, BamHI and XbaI or SpHI to completion and separated on an agarose gel and transfered onto a membrane (Amersham Hybond N). The blot was probed with DNA fragments representing the 10 kD zein chimeric gene or the selectable marker gene (Random-Primed Labeling kit from Boehringer Mannheim; hybridization following Khandjian, Bio/Technol., 5:165-167 (1987)). Figure 4 shows the gene structure of CW189 and the Southern data obtained for plant CW189-12(2). Segregation analysis for the marker gene neomycin phosphotransferase II indicated two single loci of insertion for the selectable marker gene. The complex banding pattern for plant CW189-12(2) indicated, that there are at least 2 major loci with multiple tandem insertions of the 10 kD zein gene, rearrangements and deletions. In part, the tandem insertions and most likely the multiple copies are the cause of this plant being a high expressor. The copy number for the 10 kD zein gene in plant CW 189-12(2) is estimated at approximately 25.

### EXAMPLE 8

### Transformation of Soybean with a β-Phaseolin-10 kD Zein Chimeric Gene

To induce somatic embryos, cotyledons, 4-5 mm in length dissected from surface sterilized, immature seeds of soybean, were cultured in the dark at 25°C on an agar medium (SB1 or SB2) for 8-10 weeks. Somatic embryos, which produce secondary embryos were excised and placed into a liquid medium (SB55). After repeated selection for clusters of somatic embryos which multiply as early, globular staged embryos, the suspensions were maintained as described below.

Soybean embryogenic suspension cultures were maintained in 35 mL liquid media (SB55) on a rotary shaker, 150 rpm, at 28°C with mixed florescent and incandescent lights on a 16:8 hour day/night schedule. Cultures were subcultured every two weeks.

Soybean embryogenic suspension cultures were transformed by the method of particle gun bombardment (Klein et al. (1987) Nature (London) 327:70, U.S. Patent No. 4,945,050). A Du Pont Biolistic, PDS1000/HE instrument (helium retrofit) was used for these transformations.

The plasmid vector used for transformation will be a derivative of pGEM9Z (Promega Biological Research Products). As a selectable marker a chimeric gene composed of the 35S promoter from Cauliflower Mosaic Virus [Odell et al.(1985) Nature 313:810-812], the hygromycin phosphotransferase (hygro)gene from plasmid pJR225 (from E. coli) [Gritz et al. (1983) Gene 25:179-188] and the 3' region of the nopaline synthase gene from the T-DNA of the Ti plasmid of Agrobacterium tumefaciens (see SEQ ID NO:26 in BB-1027-A) was at the Sal I site of the vector. The β-phaseolin-10 kD zein chimeric gene cassette, β-phaseolin 5' region/10 kD zein/β-phaseolin 3' region, (see pCW126 in Example 3) was isolated as an approximately 2.2 kb Hind III fragment. This fragment was inserted into the unique Hind III site of the vector resulting in plasmid pCW157.

To 50 µL of a 60 mg/mL 1 mm gold particle suspension the following were added (in order); 5 µL DNA(1 µg/µL), 20 µl spermidine (0.1 M), and 50 µL CaCl₂ (2.5 M). The particle preparation was agitated for three minutes, spun in a microfuge for 10 seconds and the supernatant was removed. The DNA-coated particles were washed once in 400 µL 70% ethanol and resuspended in 40 µL of anhydrous ethanol. The DNA/particle suspension was sonicated three times for one second each. Five µL of the DNA-coated gold particles were be loaded on each macro carrier disk.

Approximately 400-800 mg of a two-week-old suspension culture were placed in an empty 60x15 mm petri dish and the residual liquid removed from the tissue with a pipette. For each transformation experiment, approximately 5-10 plates of tissue were normally bombarded. Membrane rupture pressure was set at 1000 psi and the chamber was evacuated to a vacuum of 28 inches mercury. The tissue was placed approximately 3.5 inches away from the retaining screen and bombarded three times. Following bombardment, the tissue was placed back into liquid and cultured as described above.

Seven to fourteen days post bombardment, the liquid media will be exchanged with fresh SB55 containing 50 mg/mL hygromycin. The selective media will be refreshed weekly. Five to seven weeks post bombardment, green, transformed tissue will be observed growing from untransformed, necrotic embryogenic clusters. Isolated green tissue will be removed and inoculated into multiwell plates to generate new, clonally propagated, transformed embryogenic suspension cultures. Thus each new line will be treated as an independent transformation event. These suspensions can then be subcultured and maintained as clusters of immature embryos or regenerated into whole plants by maturation and germination of individual somatic embryos.

Transformed embryogenic clusters are removed from liquid culture and placed on a solid agar media (SB103) containing no hormones or antibiotics. Embryos are cultured for eight weeks at 26°C with mixed florescent and incandescent lights on a 16:8 hour day/night schedule. During this period, individual embryos will be removed from the clusters and analyzed for production of the 10 kD zein protein as described below. After eight weeks, the embryos are suitable for germination.

Individual embryos are frozen in liquid nitrogen, and ground to a fine powder with a morter and pestle prechilled in liquid nitrogen. The powder is scraped into an eppendorf centrifuge tube and extracted twice with hexane at room temperature. The residue is incubated at 60°C for 30 min to allow residual hexane to evaporate. Then 100 µL of 50 mM Tris-HCl pH 6.7, 2 mM EDTA, 1% SDS 1% beta-mercaptoethanol (TESβ) is added to the pellet and it is ground at low speed for about 10 sec using a motorized grinder with disposable plastic shafts designed to fit into the microfuge tube. The resultant suspensions are centrifuged for 5 min at room temperature in a microfuge and the supernatant is removed and saved. The pellet is resuspended in 50 µL of 70% isopropanol, 10mM β-mercaptoethanol by grinding as above. The tube is incubated at 60°C for 5 min and centrifuged as above. The supernatant is saved and the pellet extracted again with 50 µL of 70% isopropanol, 10 mM β-mercaptoethanol. The alcohol extracts are pooled and lyophilized; the residue is resuspended in 50 µL of TESβ. This sample and the first TESβ extract is assayed for the presence of 10 kD zein protein by Western blot as described in Example 5.

### Media:

| SB55 Stock Solutions (grams per liter): | | | |
|---|---|---|---|
| MS Sulfate 100X Stock | | MS Halides 100X Stock | |
| MgSO₄ 7H₂O | 37.0, | CaCl₂ 2H₂O | 44.0, |
| MnSO₄ H₂O | 1.69, | KI | 0.083, |
| ZnSO₄ 7H₂O | 0.86, | CoCl₂ 6H₂O | 0.00125 |
| CuSO₄ 5H₂O | 0.0025 | | |

| MS P.B.Mo 100X Stock | | MS FeEDTA 100X Stock | |
|---|---|---|---|
| KH₂PO₄ | 17.0, | Na₂EDTA | 3.724, |
| H₃BO₃ | 0.62, | FeSO₄ 7H₂O | 2.784 |
| Na₂MoO₄ 2H₂O | 0.025 | | |

| B5 Vitamin Stock | | SB55 (per liter) | |
|---|---|---|---|
| 10 g m-inositol, | | 10 mL each MS stocks, | |
| 100 mg nicotinic acid, | | 1 mL B5 Vitamin stock | |
| 100 mg pyridoxine HCl, | | 0.8 g NH₄NO₃ | |
| 1 g thiamine | | 3.033 g KNO₃ | |
| | | 1 mL 2,4-D (10 mg/mL stock) | |
| | | 60 g sucrose | |
| | | 0.667 g asparagine | |
| | | pH 5.7 | |

| SB 103 (per liter) | | SB1 (per liter) | |
|---|---|---|---|
| MS Salts | | MS Salts | |
| 6% maltose | | B5 Vitamins | |
| 750 mg MgCl₂ | | 0.175 M glucose | |
| 0.2% GELRITE | | 20 mg 2,4-D | |
| pH 5.7 0.8% agar | | | |
| | | pH 5.8 | |
| | | | |

| SB2 | | | |
|---|---|---|---|
| same as SB1 except 40 mg/L 2,4-D | | | |

## Claims

1. A chimeric gene comprising the following elements operably linked in a 5' to 3' orientation; (a) a dicotyledonous seed-specific promoter, (b) an intracellular localisation sequence, (c) the maize 10 kD zein coding region without the addition of an ER retention signal, and (d) a 3' non-coding sequence, wherein said chimeric gene causes an increase in total seed methionine in plants in which the gene is expressed as compared to untransformed plant cells; with the proviso that said seed-specific promoter is not the glycinin promoter.

2. The chimeric gene of claim 1 wherein said seed specific promoter is selected from bean β-phaseolin, bean lectin, soybean lectin, soybean kunitz trypsin inhibitor, soybean β-conglycinin, pea vicilin, pea convicilin, pea legumin, rapeseed napin, and Arabidopsis thaliana 2S seed storage protein promoters.

3. The chimeric gene of claim 1 or claim 2 capable of causing an increase in total seed methionine in rapeseed of from 29% to 232% as compared to untransformed rapeseed *Brassica napus* (cv. Westar), with 0.76g methionine per 100g of defatted seed meal.

4. The chimeric gene of any one of the preceding claims wherein the seed-specific promoter is the bean β-phaseolin promoter.

5. The chimeric gene of claim 4 wherein the bean β-phaseolin promoter comprises the bean phaseolin 5' untranslated leader region and 410 bp of sequence located immediately upstream of the transcription initiation site.

6. The chimeric gene of any one of the preceding claims wherein the 3' non-coding sequence is bean β-phaseolin 3' non-coding sequence.

7. The chimeric gene of any one of the preceding claims wherein the intracellular localisation sequence is an amino-terminal signal peptide sequence.

8. The chimeric gene of claim 7 wherein the intracellular localisation sequence is the 10 kD zein amino-terminal signal peptide sequence.

9. A dicotyledonous plant transformed with a chimeric gene comprising the following elements operably linked in a 5' to 3' orientation; (a) a dicotyledonous seed-specific promoter, (b) an intracellular localisation sequence, (c) the maize 10 kD zein coding region without the addition of an ER retention signal, and (d) a 3' non-coding sequence, wherein said chimeric gene causes an increase in total seed methionine in plants in which the gene is expressed as compared to untransformed plant cells.

10. The plant of claim 9 wherein said seed specific promoter is selected from bean β-phaseolin, bean lectin, soybean lectin, soybean kunitz trypsin inhibitor, soybean β-conglycinin, pea vicilin, pea convicilin, pea legumin, rapeseed napin, and Arabidopsis thaliana 2S seed storage protein promoters.

11. The transformed dicotyledonous plant of claim 9 or 10 wherein the plant is rapeseed.

12. The transformed dicotyledonous plant of claim 9 or 10 wherein the plant is soybean.

13. Seeds and fractions thereof comprising a chimeric gene comprising the following elements operably linked in a 5' to 3' orientation; (a) a dicotyledonous seed-specific promoter, (b) an intracellular localisation sequence, (c) the maize 10 kD zein coding region without the addition of an ER retention signal, and (d) a 3' non-coding sequence.

14. The seeds or fractions of claim 13 wherein said seed specific promoter is selected from bean β-phaseolin, bean lectin, soybean lectin, soybean kunitz trypsin inhibitor, soybean β-conglycinin, pea vicilin, pea convicilin, pea legumin, rapeseed napin, and Arabidopsis thaliana 2S seed storage protein promoters.

15. A plant according to claim 9 comprising multiple copies of the chimeric gene inserted in its genome.

16. A method of increasing the sulfur amino acid content of a dicotyledonous plant comprising:
(a) transforming a dicotyledonous plant cell with a chimeric gene comprising the following elements operably linked in a 5' to 3' orientation; (a) a dicotyledonous seed-specific promoter, (b) an intracellular localisation sequence, (c) the maize 10 kD zein coding region without the addition of an ER retention signal, and (d) a 3' non-coding sequence;
(b) growing fertile, sexually mature plants from the transformed plant cell of step (a); and
(c) selecting progeny seed from the fertile plants of step (b) for increased levels of total seed methionine relative to dicotyledonous plants of the same type as those transformed in step (a) but not containing the chimeric gene.

17. The method of claim 16 wherein said seed specific promoter is selected from bean β-phaseolin, bean lectin, soybean lectin, soybean kunitz trypsin inhibitor, soybean β-conglycinin, pea vicilin, pea convicilin, pea legumin, rapeseed napin, and Arabidopsis thaliana 2S seed storage protein promoters.

18. A method according to claim 16 or 17 wherein the plant is rapeseed and the increase in total seed methionine relative to the total seed methionine of an untransformed plant is between 29% and 232% as compared with *Brassica napus* (cv. Westar) as untransformed control with 0.76 grams methionine per 100 grams of defatted seed meal.

19. A method according to claim 18 wherein the plant is rapeseed and the seeds contain between 1% and 8% of total extractable seed protein as 10 kD zein.

20. A plant cell transformed with the chimeric gene of any one of claims 1 to 8.

21. Feed comprising fractions of the seed of claim 13 or 14.

## Patentansprüche

1. Chimäres Gen, umfassend die folgenden Elemente, operabel in einer 5' bis 3' Orientierung gekoppelt, (a) einen Zweikeimblattsamen-spezifischen Promotor, (b) eine intrazelluläre Lokalisierungssequenz, (c) die Mais 10 kD Zeincodierungsregion ohne die Zugabe eines ER Retentionssignals und (d) eine 3' Nicht-Codierungssequenz, wobei das chimäre Gen einen Anstieg an Gesamtsamenmethionin in Pflanzen erzeugt, in denen das Gen exprimiert wird, im Vergleich zu nicht transformierten Pflanzenzellen, unter der Voraussetzung, daß der Samen-spezifische Promotor nicht der Glycininpromotor ist.

2. Chimäres Gen nach Anspruch 1, wobei der Samen-spezifische Promotor ausgewählt ist aus Bohnen-β-Phaseolin, Bohnen-Lectin, Sojabohnen-Lectin, Sojabohnen-Kunitz-Trypsin-Inhibitor, Sojabohnen β-Conglycinin, Erbsen-Vicilin, Erbsen-Convicilin, Erbsen-Legumin, Rapssamen-Napin und Arabidopsis thaliana 2S Samenlagerproteinpromotoren.

3. Chimäres Gen nach Anspruch 1 oder Anspruch 2, fähig zum Erzeugen eines Anstiegs an Gesamtsamenmethionin in Rapssamen von 29% bis 232% im Vergleich zu nicht transformiertem Rapssamen *Brassica napus* (cv. Westar), mit 0,76 g Methionin pro 100 g entfettetem Sahmenmehl.

4. Chimäres Gen nach einem der vorhergehenden Ansprüche, wobei der Samen-spezifische Promotor der Bohnen β-Phaseolin Promotor ist.

5. Chimäres Gen nach Anspruch 4, wobei der Bohnen β-Phaseolin Promotor die Bohnenphaseolin 5' nicht translatierte Leaderregion und 410 bp von Sequenz, unmittelbar stromaufwärts der Transcriptionsinitiierungsstelle angeordnet, umfaßt.

6. Chimäres Gen nach einem der vorhergehenden Ansprüche, wobei die 3' Nicht-Codierungssequenz Bohnen β-Phaseolin 3' Nicht-Codierungssequenz ist.

7. Chimäres Gen nach einem der vorhergehenden Ansprüche, wobei die intrazelluläre Lokalisierungssequenz eine amino-terminale Signalpeptidsequenz ist.

8. Chimäres Gen nach Anspruch 7, wobei die intrazelluläre Lokalisierungssequenz die 10 kD Zein amino-terminale Signalpeptidsequenz ist.

9. Zweikeimblattpflanze, transformiert mit einem chimären Gen, umfassend die folgenden Elemente, operabel gekoppelt in einer 5' bis 3' Orientierung, (a) einen Zweikeimblattsamen-spezifischen Promotor, (b) eine intrazelluläre Lokalisierungssequenz, (c) die Mais 10 kD Zeincodierungsregion ohne die Zugabe eines ER Retentionssignals und (d) eine 3' Nicht-Codierungssequenz, wobei das chimäre Gen einen Anstieg an Gesamtsamenmethionin in Pflanzen erzeugt, in denen das Gen exprimiert wird, im Vergleich zu nicht-transformierten Pflanzenzellen.

10. Pflanze nach Anspruch 9, wobei der Samen spezifische Promotor aus Bohnen-β-Phaseolin, Bohnen-Lectin, Sojabohnen-Lectin, Sojabohnen-Kunitz-Trypsin-Inhibitor, Sojabohnen-β-Conglycinin, Erbsen-Vicilin, Erbsen-Convicilin, Erbsen-Legumin, Rapssamen-Napin und Arabidopsis thaliana 2S Samenlagerproteinpromotoren ausgewählt ist.

11. Transformierte Zweikeimblattpflanze nach Anspruch 9 oder 10, wobei die Pflanze Rapssamen ist.

12. Transformierte Zweikeimblattpflanze nach Anspruch 9 oder 10, wobei die Pflanze Sojabohne ist.

13. Samen oder Fraktionen davon, umfassend ein chimäres Gen, umfassend die folgenden Elemente, operabel gekoppelt in einer 5' bis 3' Orientierung, (a) einen Zweikeimblattsamen-spezifischen Promotor, (b) eine intrazelluläre Lokalisierungssequenz, (c) die Mais 10 kD Zeincodierungsregion ohne die Zugabe eines ER Retentionssignals und (d) eine 3' Nicht-Codierungssequenz.

14. Samen oder Fraktionen nach Anspruch 13, wobei der Samen spezifische Promotor ausgewählt ist aus Bohnen β-Phaseolin, Bohnen-Lectin, Sojabohnen-Lectin, Sojabohnen-Kunitz-Trypsin-Inhibitor, Sojabohnen β-Conglycinin, Erbsen-Vicilin, Erbsen-Convicilin, Erbsen-Legumin, Rapssamen-Napin und Arabidopsis thaliana 2S Samenlagerproteinpromotoren.

15. Pflanze nach Anspruch 9, umfassend mehrfache Kopien des chimären Gens, eingefügt in ihr Genom.

16. Verfahren zum Erhöhen des Schwefelaminosäurengehalts einer Zweikeimblattpflanze, umfassend:
(a) Transformieren einer Zweikeimblattpflanzenzelle mit einem chimären Gen, umfassend die folgenden Elemente, operabel in einer 5' bis 3' Orientierung gekoppelt, (a) einen Zweikeimblattsamen spezifischen Promotor, (b) eine intrazelluläre Lokalisierungssequenz, (c) die Mais 10 kD Zeincodierungsregion ohne die Zugabe eines ER Retentionssignals und (d) eine 3' Nicht-Codierungssequenz,
(b) Züchten fruchtbarer, sexuell reifer Pflanzen aus der transformierten Pflanzenzelle von Stufe (a) und
(c) Auswählen von Nachkommenschaftsamen aus den fruchtbaren Pflanzen von Stufe (b) für erhöhte Spiegel von Gesamtsamenmethionin relativ zu Zweikeimblattpflanzen des gleichen Typs wie diejenigen, transformiert in Stufe (a), aber nicht das chimäre Gen enthaltend.

17. Verfahren nach Anspruch 16, wobei der Samen spezifische Promotor aus Bohnen β-Phaseolin, Bohnen-Lectin, Sojabohnen-Lectin, Sojabohnen-Kunitz-Trypsin-Inhibitor, Sojabohnen β-Conglycinin, Erbsen-Vicilin, Erbsen-Convicilin, Erbsen-Legumin, Rapssamen-Napin und Arabidopsis thaliana 2S Samenlagerproteinpromotoren ausgewählt wird.

18. Verfahren nach Anspruch 16 oder 17, wobei die Pflanze Rapssamen ist, und der Anstieg an Gesamtsamenmethionin relativ zu dem Gesamtsamenmethionin einer nicht transformierten Pflanze ist zwischen 29% und 232% im Vergleich zu *Brassica napus* (cv. Westar) als nicht transformierte Kontrolle, mit 0,76 Gramm Methionin pro 100 Gramm entfettetem Sahmenmehl.

19. Verfahren nach Anspruch 18, wobei die Pflanze Rapssamen ist, und die Samen enthalten zwischen 1% und 8% gesamtes extrahierbares Samenprotein als 10 kD Zein.

20. Pflanzenzelle, transformiert mit dem chimären Gen nach einem von Ansprüchen 1 bis 8.

21. Nahrung, umfassend Fraktionen des Samens nach Anspruch 13 oder 14.

## Revendications

1. Gène chimérique comprenant les éléments suivants liés de manière active dans une orientation de 5' à 3'; (a) un promoteur spécifique à une semence de dicotylédone, (b) une séquence de localisation intracellulaire, (c) la région codant pour la 10 kD zéine du maïs sans l'ajout d'un signal de rétention ER, et (d) une séquence non codante 3', dans lequel ledit gène chimérique entraîne une augmentation de la méthionine totale de semences dans des plantes dans lesquelles le gène est exprimé, comparé à des cellules de plantes non transformées; à condition que ledit promoteur spécifique à une semence ne soit pas le promoteur de glycinine.

2. Gène chimérique suivant la revendication 1, dans lequel ledit promoteur spécifique à une semence est choisi parmi la β-phaséoline de haricot, la lectine de haricot, la lectine de soja, l'inhibiteur de trypsine de type Kunitz de soja, la β-conglycinine de soja, la viciline de pois, la conviciline de pois, la légumine de pois, la napine de colza et des promoteurs de protéines de stockage de semence d'Arabidopsis thaliana 2S.

3. Gène chimérique suivant la revendication 1 ou la revendication 2, capable d'entraîner une augmentation dans la méthionine totale de semences dans du colza de 29% à 232%, comparé à un *Brassica napus* de colza non transformé (cv. Westar), avec 0,76 g de méthionine pour 100 g de farine de semence dégraissée.

4. Gène chimérique suivant l'une quelconque des revendications précédentes, dans lequel le promoteur spécifique à une semence est le promoteur de β-phaséoline de haricot.

5. Gène chimérique suivant la revendication 4, dans lequel le promoteur de β-phaséoline de haricot comprend la région de tête non traduite 5' de phaséoline de haricot et 410 bp de séquence située immédiatement en amont du site d'initiation de transcription.

6. Gène chimérique suivant l'une quelconque des revendications précédentes, dans lequel la séquence non codante 3' est la séquence non codante 3' de la β-phaséoline de haricot.

7. Gène chimérique suivant l'une quelconque des revendications précédentes, dans lequel la séquence de localisation intracellulaire est une séquence de peptide signal à extrémité amino.

8. Gène chimérique suivant la revendication 7, dans lequel la séquence de localisation intracellulaire est la séquence de peptide signal à extrémité amino de la 10 kD zéine.

9. Plante dicotylédone transformée avec un gène chimérique comprenant les éléments suivants liés de manière active dans une orientation de 5' à 3'; (a) un promoteur spécifique à une semence de dicotylédone, (b) une séquence de localisation intracellulaire, (c) la région codant pour la 10 kD zéine du maïs sans l'ajout d'un signal de rétention ER, et (d) une séquence non codante 3', dans laquelle ledit gène chimérique entraîne une augmentation de la méthionine totale de semences dans des plantes dans lesquelles le gène est exprimé, comparé à des cellules de plantes non transformées.

10. Plante suivant la revendication 9, dans laquelle ledit promoteur spécifique à une semence est choisi parmi la β-phaséoline de haricot, la lectine de haricot, la lectine de soja, l'inhibiteur de trypsine de type Kunitz de soja, la β-conglycinine de soja, la viciline de pois, la conviciline de pois, la légumine de pois, la napine de colza et des promoteurs de protéines de stockage de semence d'Arabidopsis thaliana 2S.

11. Plante dicotylédone transformée suivant la revendication 9 ou 10, dans laquelle la plante est du colza.

12. Plante dicotylédone transformée suivant la revendication 9 ou 10, dans laquelle la plante est du soja.

13. Semences et fractions de celles-ci comprenant un gène chimérique comprenant les éléments suivants liés de manière active dans une orientation de 5' à 3'; (a) un promoteur spécifique à une semence de dicotylédone, (b) une séquence de localisation intracellulaire, (c) la région codant pour la 10 kD zéine du maïs sans l'ajout d'un signal de rétention ER, et (d) une séquence non codante 3'.

14. Semences ou fractions suivant la revendication 13, dans lesquelles ledit promoteur spécifique à une semence est choisi parmi la β-phaséoline de haricot, la lectine de haricot, la lectine de soja, l'inhibiteur de trypsine de type Kunitz de soja, la β-conglycinine de soja, la viciline de pois, la conviciline de pois, la légumine de pois, la napine de colza et des promoteurs de protéines de stockage de semence d'Arabidopsis thaliana 2S.

15. Plante suivant la revendication 9, comprenant de multiples copies du gène chimérique inséré dans son génome.

16. Procédé pour l'augmentation de la teneur d'acides aminés sulfureux d'une plante dicotylédone comprenant:
(a) la transformation d'une cellule de plante dicotylédone avec un gène chimérique comprenant les éléments suivants liés de manière active dans une orientation de 5' à 3'; (a) un promoteur spécifique à une semence de dicotylédone, (b) une séquence de localisation intracellulaire, (c) la région codant pour la 10 kD zéine du maïs sans l'ajout d'un signal de rétention ER, et (d) une séquence non codante 3';
(b) la croissance de plantes fertiles, sexuellement matures à partir de la cellule de plante transformée de l'étape (a); et
(c) la sélection d'une semence de descendance à partir des plantes fertiles de l'étape (b) pour des niveaux accrus de méthionine totale de semences par rapport à ceux de plantes dicotylédones du même type que celles qui sont transformées dans l'étape (a) mais ne contenant pas le gène chimérique.

17. Procédé suivant la revendication 16, dans lequel ledit promoteur spécifique à une semence est choisi parmi la β-phaséoline de haricot, la lectine de haricot, la lectine de soja, l'inhibiteur de trypsine de type Kunitz de soja, la β-conglycinine de soja, la viciline de pois, la conviciline de pois, la légumine de pois, la napine de colza et des promoteurs de protéines de stockage de semence d'Arabidopsis thaliana 2S.

18. Procédé suivant la revendication 16 ou 17, dans lequel la plante est du colza et l'augmentation de la méthionine totale de semences par rapport à la méthionine totale de semences d'une plante non transformée est entre 29% et 232%, comparé à un *Brassica napus* (cv. Westar) en tant que témoin non transformé avec 0,76 grammes de méthionine pour 100 grammes de farine de semence dégraissée.

19. Procédé suivant la revendication 18, dans lequel la plante est du colza et les semences contiennent entre 1% et 8% de protéine de semence pouvant être extraite totale sous forme de 10 kD zéine.

20. Cellule de plante transformée avec le gène chimérique suivant l'une quelconque des revendications 1 à 8.

21. Aliment comprenant des fractions de la semence suivant la revendication 13 ou 14.
